# EUROPEAN PATENT SPECIFICATION

(11) **EP 3 707 159 B1**
(45) Date of publication and mention of the grant of the patent: **04.01.2023**
(21) Application number: 18803349.2
(22) Date of filing: 05.11.2018
(51) Int. Cl.: A61K 39/00, A61P 35/00, C07K 16/18, C07K 14/725, C07K 14/78, C07K 16/28, C07K 16/30, C12N 5/0783, C12N 15/62

(54) **NOVEL ENGINEERED T CELL RECEPTORS AND IMMUNE THERAPY USING THE SAME**
NEUARTIGE MANIPULIERTE T-ZELL-REZEPTOREN UND IMMUNTHERAPIE DAMIT
NOUVEAUX RÉCEPTEURS DE LYMPHOCYTES T MODIFIÉS ET IMMUNOTHÉRAPIE LES UTILISANT

(30) Priority: 06.11.2017 DE 102017125888; 06.11.2017 US 201762582202 P
(43) Date of publication of application: 16.09.2020
(73) Proprietor: Immatics Biotechnologies GmbH, 72076 Tübingen (DE)
(72) Inventor: UNVERDORBEN, Felix, 70199 Stuttgart (DE); BUNK, Sebastian, 72074 Tübingen (DE); HOFMANN, Martin, 72076 Tübingen (DE); HUTT, Meike, 70565 Stuttgart (DE); MAURER, Dominik, 72116 Moessingen (DE); ALTEN, Leonie, 72076 Tübingen (DE); WAGNER, Claudia, 72074 Tübingen (DE)
(74) Representative: Zwicker, Jörk
(86) International application number: PCT/EP2018/080176
(87) International publication number: WO 2019/086665

(56) References cited:
- WO-A1-2016/156202
- WO-A1-2018/033291
- WO-A2-2011/113819
- Emma S. Hickma: "Antigen Selection for Enhanced Affinity T-Cell Receptor-Based Cancer Therapies", Journal of Biomolecular Screening Society for Laboratory Automation and Screening, 1 January 2016 (2016-01-01), pages 769-785, XP55534795, Retrieved from the Internet: URL:http://citenpl.internal.epo.org/wf/web /citenpl/citenpl.html [retrieved on 2018-12-14]

## Description

### FIELD OF THE INVENTION

The present invention pertains to antigen recognizing constructs against COL6A3 antigens. The invention in particular provides novel engineered T cell receptor (TCR) based molecules which are selective and specific for the tumor expressing antigen COL6A3. The TCR of the invention, and COL6A3 antigen binding fragments derived therefrom, are of use for the diagnosis, treatment and prevention of COL6A3 expressing cancerous diseases. Further provided are nucleic acids encoding the antigen recognizing constructs of the invention, vectors comprising these nucleic acids, recombinant cells expressing the antigen recognizing constructs and pharmaceutical compositions comprising the compounds of the invention.

### BACKGROUND

The collagens are a superfamily of proteins that play a role in maintaining the integrity of various tissues. Collagens are extracellular matrix proteins and have a triple-helical domain as their common structural element. Collagen VI is a major structural component of micro fibrils. The basic structural unit of collagen VI is a heterotrimer of the alpha l(VI), alpha 2(VI), and alpha 3(VI) collagen chains. The alpha l(VI) and alpha 2(VI) chains are encoded by the COL6A1 and COL6A2 genes, respectively. The protein encoded by the COL6A3 gene is the alpha 3 subunit of type VI collagen (alpha 3(VI) collagen chain) (Bertini et al., 2002 Eur. J. Paediatr. Neurol 6:193-8). COL6A3's gene expression was previously shown to be associated with the progression of breast cancer and was elevated in colon cancer (Smith MJ, et al. "Analysis of differential gene expression in colorectal cancer and stroma using fluorescence-activated cell sorting purification" British journal of cancer. 2009;100:1452-1464; Tilman G et al "Human periostin gene expression in normal tissues, tumors and melanoma: evidences for periostin production by both stromal and melanoma cells" Mol Cancer. 2007;6:80) and as a prognosis marker of colorectal carcinoma (Qiao J et al. "Stroma derived COL6A3 is a potential prognosis marker of colorectal carcinoma revealed by quantitative proteomics" Oncotarget. 2015 Oct 6; 6(30): 29929-29946). COL6A3 gene locates 2q37 in the human genome and contains 44 exons. The COL6A3 protein has 3177 amino acids and contains 12 Von Willebrand factor type A (vWA) domains, one fibronectin type 3 domain and one BPTI/Kunitz family of serine protease inhibitors (KU) domain.

Targets for T-cell based immunotherapy represent peptide epitopes derived from tumor-associated or tumor-specific proteins, which are presented by molecules of the major histocompatibility complex (MHC). These tumor associated antigens (TAAs) can be peptides derived from all protein classes, such as enzymes, receptors, transcription factors, etc. which are expressed and, as compared to unaltered cells of the same origin, usually up-regulated in cells of the respective tumor.

Specific elements of the cellular immune response are capable of selectively recognizing and destroying tumor cells. The isolation of tumor antigen-specific T cells from tumor-infiltrating cell populations or from peripheral blood suggests that such cells play an important role in natural immune defense against cancer. CD8-positive T cells in particular, which recognize class I molecules of the major histocompatibility complex (MHC)-bearing peptides of usually 8 to 10 amino acid residues derived from proteins or defective ribosomal products (DRiPs) located in the cytosol, play an important role in this response. The MHC-molecules of the human are also designated as human leukocyte-antigens (HLA).

A TCR is a heterodimeric cell surface protein of the immunoglobulin super-family, which is associated with invariant proteins of the CD3 complex involved in mediating signal transduction. TCRs exist as αβ and γδ heterodimers, which are structurally similar but have quite distinct anatomical locations and probably functions. The extracellular portion of native heterodimeric αβTCR consists of two polypeptide chains, each of which has a membrane-proximal constant domain, and a membrane-distal variable domain. Each of the constant and variable domains includes an intra-chain disulfide bond. The variable domains contain the highly polymorphic loops analogous to the complementarity determining regions (CDRs) of antibodies. The use of TCR gene therapy overcomes a number of current hurdles. It allows equipping patients' own T cells with desired specificities and generation of sufficient numbers of T cells in a short period of time, avoiding their exhaustion. The TCR will be transduced into central memory T cells or T cells with stem cell characteristics, which may ensure better persistence and function upon transfer. TCR-engineered T cells will be infused into cancer patients rendered lymphopenic by chemotherapy or irradiation, allowing efficient engraftment but inhibiting immune suppression.

WO 2011/113819 describes T-cells recognizing tumor associated antigens, but binding thereto with low affinity and limited specificity. Similar, WO 2016/156202 relates to tumor associated antigens and T cell receptors binding thereto.

While advances have been made in the development of molecular-targeting drugs for cancer therapy, there remains a need in the art to develop new anti-cancer agents that specifically target molecules highly specific to cancer cells. The present description addresses that need by providing novel engineered COL6A3 TCRs, respective recombinant TCR constructs, nucleic acids, vectors and host cells that specifically bind COL6A3 epitope(s) as disclosed; and methods of using such molecules in the treatment of cancer.

### DESCRIPTION

The invention is defined by the appended claims.

In a first aspect the object of the invention is solved by an antigen recognizing construct comprising a first domain comprising three complementary determining regions (CDRs) according to SEQ ID NOs: 6 (CDRa2), and 7 (CDRa3), and a second domain comprising three complementary determining regions (CDRs) according to SEQ ID NOs: 14 (CDRb2), and 15 (CDRb3), wherein
a) the CDRal of the first domain is as set forth in SEQ ID NO:5 and the CDRbl of the second domain is selected from one of the amino acid sequences according to SEQ ID NOs: 37 to 44, or
b) the CDRal of the first domain is as set forth in SEQ ID NO:26 and the CDRbl of the second domain is selected from one of the amino acid sequences according to SEQ ID NOs: 13 and 37 to 49, wherein the antigen recognizing construct is a T cell receptor (TCR) or derivative or fragment thereof, wherein the derivative or fragment retains the antigen binding and/or recognizing ability of the molecule to specifically and/or selectively bind to a COL6A3 antigenic polypeptide.

In another disclosure of the present teaching not being covered by the claims, CDRal of an antigen recognizing construct may have at least about 25%, at least about 30%, at least about 35%, at least about 40%, at least about 45%, at least about 50%, at least about 55%, at least about 60%, at least 65%, at least about 70%, at least about 75%, at least about 80%, at least about 85%, at least about 90%, at least about 95%, at least about 96%, at least about 97%, at least about 98%, or at least about 99% identity with the amino acid sequence according to SEQ ID NO: 5.

In another disclosure of the present teaching not being covered by the claims , CDRa2 of an antigen recognizing construct may have at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity with the amino acid sequence according to SEQ ID NO: 6.

In another disclosure of the present teaching not being covered by the claims , CDRa3 of an antigen recognizing construct may have at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity with the amino acid sequence according to SEQ ID NO: 7.

In another disclosure of the present teaching not being covered by the claims , CDRb1 of an antigen recognizing construct may have at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity with the amino acid sequence according to SEQ ID NO: 13.

In another disclosure of the present teaching not being covered by the claims , CDRb2 of an antigen recognizing construct may have at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity with the amino acid sequence according to SEQ ID NO: 14.

In another disclosure of the present teaching not being covered by the claims , CDRb3 of an antigen recognizing construct may have at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity with the amino acid sequence according to SEQ ID NO: 15.

In the context of the present invention, the inventors have identified improved engineered versions of the COL6A3 TCR R4P3F9 comprising mutated CDR1 sequences in the alpha and beta chain(s) that are improving stability, recognition and selectivity of the parental R4P3F9. These maturated TCR variants have been selected in a two-step method, wherein one step selects for stability and the second for affinity of the variants (see examples). While the inventive TCRs comprise mutated CDR1 regions, it is likely that CDR2 and or CDR3 can be mutated as well in order to increase binding affinity/specificity and/or selectivity and such mutated CDRs ideally could be included in the existing constructs.

The affinity maturation identified variants with considerably stronger binding activity towards HLA-A^{∗}02/COL6A3-peptide, while retaining or even improving specificity.

Compared to the parental TCR C-1 (R4P3F9 TCR comprising wild type CDRs, see Table 5), all variants of the invention improved IFN-gamma release with higher levels reached already for lower peptide loading concentrations.

Within the variable domain, CDR1 and CDR2 are found in the variable (V) region of a polypeptide chain, and CDR3 includes some of V, all of diversity (D) and joining (J) regions. It is assumed that CDR3 is the most variable and is the main CDR responsible for specifically and selectively recognizing an antigen. Surprisingly, for some TCRs CDR1 seems also to make contacts to the peptide and thus is also responsible for selective recognition. In the present case, without being bound to a specific theory, the mutated CDR1b seems to interact with position 8 of the COL6A3-peptide.

Native alpha-beta heterodimeric TCRs have an alpha chain and a beta chain. Each alpha chain comprises variable, joining and constant regions, and the beta chain also usually contains a short diversity region between the variable and joining regions, but this diversity region is often considered as part of the joining region. Each variable region comprises three CDRs (Complementarity Determining Regions) embedded in a framework sequence, one being the hypervariable region named CDR3. There are several types of alpha chain variable (Vα) regions and several types of beta chain variable (Vβ) regions distinguished by their framework, CDR1 and CDR2 sequences, and by a partly defined CDR3 sequence. The Vα types are referred to in IMGT nomenclature by a unique TRAV number, Vβ types are referred to by a unique TRBV number.

Preferably, an antigen recognizing construct of the invention comprises the respective CDR1 to CDR3 of one individual herein disclosed engineered TCR variable region of the invention. Preferred are antigen recognizing constructs (e.g. αβ and γδ TCRs) of the invention which comprise at least one, preferably two, maturated CDR1 sequences.

The CDR-variants as taught herein - in particular the CDR1-variants - can be modified by the substitution of one or more residues at different, possibly selective, sites within the peptide chain, if not otherwise stated. Such substitutions are of a conservative nature where one amino acid is replaced by an amino acid of similar structure and characteristics, such as where a hydrophobic amino acid is replaced by another hydrophobic amino acid.

Even more conservative would be replacement of amino acids of the same or similar size and chemical nature, such as where leucine is replaced by isoleucine. In studies of sequence variations in families of naturally occurring homologous proteins, certain amino acid substitutions are more often tolerated than others, and these are often show correlation with similarities in size, charge, polarity, and hydrophobicity between the original amino acid and its replacement, and such is the basis for defining "conservative substitutions". Preferred conservative substitutions taught herein are defined as exchanges within one of the following five groups: Group 1-small aliphatic, nonpolar or slightly polar residues (Ala, Ser, Thr, Pro, Gly); Group 2-polar, negatively charged residues and their amides (Asp, Asn, Glu, Gln); Group 3-polar, positively charged residues (His, Arg, Lys); Group 4-large, aliphatic, nonpolar residues (Met, Leu, Ile, Val, Cys); and Group 5-large, aromatic residues (Phe, Tyr, Trp).

Less conservative substitutions might involve the replacement of one amino acid by another that has similar characteristics but is somewhat different in size, such as replacement of an alanine by an isoleucine residue.

The term "specificity" or "antigen specificity" or "specific for" a given antigen, as used herein means that the antigen recognizing construct can specifically bind to said antigen, a COL6A3 antigen, preferably with high avidity, when said antigen is presented by HLA, preferably HLA A2. For example, a TCR, as antigen recognizing construct, may be considered to have "antigenic specificity" for COL6A3 antigens, if T cells expressing the TCR in response to COL6A3 presenting HLA secrete at least about 200 pg/ml or more (e.g., 250 pg/ml or more, 300 pg/ml or more, 400 pg/ml or more, 500 pg/ml or more, 600 pg/ml or more, 700 pg/ml or more, 1000 pg ml or more, 2,000 pg/ml or more, 2,500 pg/ml or more, 5,000 pg/ml or more) of interferon γ (IFN-γ) upon co-culture with HLA A2 target cells pulsed with a low concentration of a COL6A3 antigen, such as the COL6A3 epitopes and antigens provided herein below (e.g., about 10-11 mol/l, 10-10 mol/l, 10-9 mol/l, 10-8 mol/l, 10-7 mol/l, 10-6 mol/l, 10-5 mol/1). Alternatively, or additionally, a TCR may be considered to have "antigenic specificity" for COL6A3, if T cells expressing the TCR secrete at least twice as much IFN-γ as the untransduced background level of IFN-γ upon co-culture with target cells pulsed with a low concentration of COL6A3 antigens. Such a "specificity" as described above can - for example - be analyzed with an ELISA.

In one alternative or additional embodiment of the invention, the antigen recognizing construct is stable, and capable of specifically and/or selectively binding to a COL6A3 antigen; preferably wherein the COL6A3 antigen is a protein epitope or peptide having an amino acid sequence shown in SEQ ID NO: 1 or a variant thereof, wherein the variant is an amino acid deletion, addition, insertion or substitution of not more than three, preferably two and most preferably not more than one amino acid position.

The term "selectivity" or "selective recognizing/binding" is understood to refer to the property of an antigen recognizing construct, such as a TCR or antibody, to selectively recognize or bind to preferably only one specific epitope and preferably shows no or substantially no cross-reactivity to another epitope. Preferably "selectivity" or "selective recognizing/binding" means that the antigen recognizing construct (e.g. a TCR) selectively recognizes or binds to preferably only one specific epitope and preferably shows no or substantially no cross-reactivity to another epitope, wherein said epitope is unique for one protein, such that the antigen recognizing construct shows no or substantially no cross-reactivity to another epitope and another protein.

The antigen recognizing construct according to the invention is a T cell receptor (TCR), or a derivative or fragment thereof. A derivative or fragment of a TCR of the invention retains the antigen binding/recognizing ability of the parent molecule, in particular its specificity and/or selectivity as explained above.

In an embodiment of the invention, the inventive engineered TCRs are able to recognize COL6A3 antigens in a major histocompatibility complex (MHC) class I-dependent manner. "MHC class I-dependent manner," as used herein, means that the TCR elicits an immune response upon binding to COL6A3 peptide antigens within the context of an MHC class I molecule. The MHC class I molecule can be any MHC class I molecule known in the art, e.g., HLA-A molecules. In a preferred embodiment of the invention, the MHC class I molecule is an HLA-A2 molecule.

The invention provides both single chain antigen recognizing construct and double chain recognizing constructs, as well as other variant molecules. The invention in particular provides an engineered TCR as antigen recognizing construct, or fragment or derivative thereof. The engineered TCR preferably is of human origin, which is understood as being generated from a human TCR locus and therefore comprising human TCR sequences. Furthermore, the TCR of the invention is characterized as affinity maturated TCR, which is capable of specifically and selectively recognizing COL6A3 peptide antigen.

Another embodiment of the invention additionally or alternatively provides the antigen recognizing construct described above, which induces an immune response, preferably wherein the immune response is characterized by an increase in interferon (IFN) γ levels.

TCRs of the invention may be provided as single chain α or β, or γ and δ, molecules, or alternatively as double chain constructs composed of both the α and β chain, or γ and δ chain.

Most preferably, in some additional embodiments, wherein the disclosure refers to antigen recognizing constructs comprising all of the CDR1 to CDR3 regions of the herein disclosed engineered TCR chains (see table 1). Antigen recognizing constructs of the present teaching may be preferred, which comprise the natural or the engineered CDR sequence having three, two, and preferably only one, modified amino acid residues. A modified amino acid residue of the present teaching may be selected from an amino acid insertion, deletion or substitution. Most preferred teaching is that the three, two, preferably one modified amino acid residue is the first or last amino acid residue of the respective CDR sequence. If the modification is a substitution then it is preferable in the present teaching that the substitution is a conservative amino acid substitution.

The inventive TCRs may further comprise a constant region derived from any suitable species, such as any mammal, e.g., human, rat, monkey, rabbit, donkey, or mouse. In an embodiment of the invention, the inventive TCRs further comprise a human constant region. In some preferred embodiments, the constant region of the TCR of the invention may be slightly modified, for example, by the introduction of heterologous sequences, preferably mouse sequences, which may increase TCR expression and stability. Also, further stabilizing mutations as known from the state of the art (e.g. DE 10 2016 123 893.7) may be introduced, such as replacement of unfavorable amino acids in the V regions and/or the introduction of a disulfide bridge between the TCR C domains and the removal of unpaired cysteine.

As used herein, the term "murine" or "human," when referring to an antigen recognizing construct, or a TCR, or any component of a TCR described herein (e.g., complementarity determining region (CDR), variable region, constant region, α chain, and/or β chain), means a TCR (or component thereof), which is derived from a mouse or a human unrearranged TCR locus, respectively.

In an embodiment of the invention, chimeric TCR are provided, wherein the TCR chains comprise sequences from multiple species. Preferably, a TCR of the invention may comprise an α chain comprising a human variable region of an α chain and, for example, a murine constant region of a murine TCR α chain.

In one embodiment, the TCR of the invention is a human TCR comprising human variable regions according to the above embodiments and human constant regions.

The TCR of the invention may be provided as a single chain TCR (scTCR). A scTCR can comprise a polypeptide of a variable region of a first TCR chain (e.g., an alpha chain) and a polypeptide of an entire (full-length) second TCR chain (e.g., a beta chain), or vice versa. Furthermore, the scTCR can optionally comprise one or more linkers which join the two or more polypeptides together. The linker can be, for instance, a peptide, which joins together two single chains, as described herein. Also provided is such a scTCR of the invention, which is fused to a human cytokine, such as IL-2, IL-7 or IL-15.

The antigen recognizing construct according to the invention can also be provided in the form of a multimeric complex, comprising at least two scTCR molecules, wherein said scTCR molecules are each fused to at least one biotin moiety, and wherein said scTCRs are interconnected by biotin-streptavidin interaction to allow the formation of said multimeric complex. Similar approaches for the generation of multimeric TCR are also possible and included in this disclosure. Also provided are multimeric complexes of a higher order, comprising more than two scTCR of the invention.

For the purposes of the present invention, a TCR is a moiety having at least one TCR alpha or gamma and/or TCR beta or delta variable domain. Generally, they comprise both a TCR alpha variable domain and a TCR beta variable domain. They may be αβ heterodimers or may be in single chain format. For use in adoptive therapy, an αβ heterodimeric TCR may, for example, be transfected as full length chains having both cytoplasmic and transmembrane domains. If desired, an introduced disulfide bond between residues of the respective constant domains may be present.

In a preferred embodiment, the antigen recognizing construct is a human TCR, or fragment or derivative thereof. A human TCR or fragment or derivative thereof is a TCR, which comprises over 50% of the corresponding human TCR sequence. Preferably, only a small part of the TCR sequence is of artificial origin or derived from other species. It is known, however, that chimeric TCRs, e.g. derived from human origin with murine sequences in the constant domains, are advantageous. Particularly preferred are, therefore, TCRs in accordance with the present invention, which contains murine sequences in the extracellular part of their constant domains.

Thus, it is also preferred that the inventive antigen recognizing construct is able to recognize its peptide antigen in a human leucocyte antigen (HLA) dependent manner, preferably in an HLA-A02 dependent manner. The term "HLA dependent manner" in the context of the present invention means that the antigen recognizing construct binds to the antigen only in the event that the antigenic peptide is presented by said HLA.

The antigen recognizing construct in accordance with the invention in one embodiment preferably induces an immune response, preferably wherein the immune response is characterized by the increase in interferon (IFN) γ levels.

The term "polypeptide" as used herein includes oligopeptides and refers to a single chain of amino acids connected by one or more peptide bonds. With respect to the inventive polypeptides, the functional portion can be any portion comprising contiguous amino acids of the TCR (or functional variant thereof), of which it is a part, provided that the functional portion specifically binds to a COL6A3 antigen, preferably as disclosed herein in table 1. The term "functional portion" when used in reference to a TCR (or functional variant thereof) refers to any part or fragment of the TCR (or functional variant thereof) of the invention, which part or fragment retains the biological activity of the TCR (or functional variant thereof), of which it is a part (the parent TCR or parent functional variant thereof). Functional portions encompass, for example, those parts of a TCR (or functional variant thereof) that retain the ability to specifically bind to a COL6A3 antigen (in an HLA-A2-dependent manner), or detect, treat, or prevent cancer, to a similar extent, the same extent, or to a higher extent, as the parent TCR (or functional variant thereof).

The functional portion can comprise additional amino acids at the amino or carboxy terminus of the portion, or at both termini, in which additional amino acids are not found in the amino acid sequence of the parent TCR or functional variant thereof. Desirably, the additional amino acids do not interfere with the biological function of the functional portion, e.g., specifically binding to COL6A3 antigens; and/or having the ability to detect cancer, treat or prevent cancer, etc. More desirably, the additional amino acids enhance the biological activity, as compared to the biological activity of the parent TCR or functional variant thereof.

Although this is not covered by the claimed invention, the binding functionality of the TCR of the invention may be provided in the framework of an antibody. The term "antibody" in its various grammatical forms is used herein to refer to immunoglobulin molecules and immunologically active portions of immunoglobulin molecules, i.e., molecules that contain an antigen-binding site or a paratope. Such molecules are also referred to as "antigen binding fragments" of immunoglobulin molecules. Further disclosed herein although not covered by the claimed invention is an antibody, or antigen binding portion thereof, which specifically binds to the antigens described herein. The antibody can be any type of immunoglobulin that is known in the art. For instance, the antibody can be of any isotype, e.g., IgA, IgD, IgE, IgG, IgM, etc. The antibody can be monoclonal or polyclonal. The antibody can be a naturally-occurring antibody, e.g., an antibody isolated and/or purified from a mammal, e.g., mouse, rabbit, goat, horse, chicken, hamster, human, etc. Alternatively, the antibody can be a genetically-engineered antibody, e.g., a humanized antibody or a chimeric antibody. The antibody can be in monomeric or polymeric form.

The above teaching applies to antigen binding portions of any of the antibodies described herein. The antigen binding portion can be any portion that has at least one antigen binding site, such as Fab, F(ab')2, dsFv, scFv, diabodies, and triabodies. A single-chain variable region fragment (scFv) antibody fragment, which consists of a truncated Fab fragment comprising the variable (V) domain of an antibody heavy chain linked to a V domain of a light antibody chain via a synthetic peptide, can be generated using routine recombinant DNA technology techniques. Similarly, disulfide-stabilized variable region fragments (dsFv) can be prepared by recombinant DNA technology, antibody fragments of the invention, however, are not limited to these exemplary types of antibody fragments. Also, the antibody, or antigen binding portion thereof, can be modified to comprise a detectable label, such as, for instance, a radioisotope, a fluorophore (e.g., fluorescein isothiocyanate (FITC), phycoerythrin (PE)), an enzyme (e.g., alkaline phosphatase, horseradish peroxidase), and element particles (e.g., gold particles).

Suitable methods of making antibodies are known in the art. For instance, standard hybridoma methods are described in, e.g., Kohler and Milstein, Eur. J. Immunol, 5, 51 1-519 (1976), Harlow and Lane (eds.), Antibodies: A Laboratory Manual, CSH Press (1988), and C.A. Janeway et al. (eds.), Immunobiology, 8 Ed., Garland Publishing, New York, NY (201 1)). Alternatively, other methods, such as EBV-hybridoma methods (Haskard and Archer, J. Immunol. Methods, 74(2), 361-67 (1984), and Roder et al, Methods Enzymol, 121, 140-67 (1986)), and bacteriophage vector expression systems (see, e.g., Huse et al., Science, 246, 1275-81 (1989)) are known in the art. Further, methods of producing antibodies in non-human animals are described in, e.g., U.S. Patents 5,545,806, 5,569,825, and 5,714,352, and U.S. Patent Application Publication No. 2002/0197266.

Some embodiments of the invention also pertain to TCRs, or functional fragments and polypeptides thereof, which are soluble TCRs. As used herein, the term "soluble T-cell receptor" refers to single chain or heterodimeric truncated variants of native TCRs, which comprise at least the variable domains of the TCR α-chain and β-chain linked by a polypeptide linker (SEQ ID NOs: 22, 24, 25 and 27). Soluble variants of TCRs usually lack at least the transmembrane and cytosolic domains of the native protein; sometimes preferably such soluble constructs do not comprise any constant domain sequences. The soluble T-cell receptor constructs of the invention, in preferred embodiments, comprise constructs consisting of α- and β-chain variable domain sequences as provided herein, connected by a suitable linker sequence. The variable domain sequence (amino acid or nucleic acid) of the soluble TCR α-chain and β-chains may be identical to the corresponding sequences in a native TCR or may comprise variants of soluble TCR α-chain and β-chain variable domain sequences, as compared to the corresponding native TCR sequences. The term "soluble T-cell receptor" as used herein encompasses soluble TCRs with variant or non-variant soluble TCR α-chain and β-chain variable domain sequences. The variations may be in the framework regions of the soluble TCR α-chain and β-chain variable domain sequences and can include, but are not limited to, amino acid deletion, insertion, substitution mutations as well as changes to the nucleic acid sequence, which do not alter the amino acid sequence. Soluble TCR of the invention in any case retain or preferentially improve the binding functionality of their parental TCR molecules.

The above problem is further solved by a nucleic acid encoding for an antigen recognizing construct of the invention, or any of the aforementioned protein or polypeptide constructs. The nucleic acid preferably (a) has a strand encoding for an antigen recognizing construct according to the invention; (b) has a strand complementary to the strand in (a); or (c) has a strand that hybridizes under stringent conditions with a molecule as described in (a) or (b). Stringent conditions are known to the person of skill in the art, specifically from Sambrook et al, "Molecular Cloning". In addition to that, the nucleic acid optionally has further sequences, which are necessary for expressing the nucleic acid sequence corresponding to the protein, specifically for expression in a mammalian/human cell. The nucleic acid used can be contained in a vector suitable for allowing expression of the nucleic acid sequence corresponding to the polypeptide in a cell. However, the nucleic acids can also be used to transform an antigen-presenting cell, which may not be restricted to classical antigen-presenting cells, such as dendritic cells, in such a way that they themselves produce the corresponding proteins on their cellular surface.

By "nucleic acid" as used herein includes "polynucleotide," "oligonucleotide," and "nucleic acid molecule," and generally means a polymer of DNA or RNA, which can be single-stranded or double-stranded, synthesized or obtained (e.g., isolated and/or purified) from natural sources, which can contain natural, non-natural or altered nucleotides, and can contain a natural, non-natural or altered internucleotide linkage, such as a phosphoroamidate linkage or a phosphorothioate linkage, instead of the phosphodiester found between the nucleotides of an unmodified oligonucleotide.

Preferably, the nucleic acids of the invention are recombinant. As used herein, the term "recombinant" refers to (i) molecules that are constructed outside living cells by joining natural or synthetic nucleic acid segments to nucleic acid molecules that can replicate in a living cell, or (ii) molecules that result from the replication of those described in (i) above. For purposes herein, the replication can be in vitro replication or in vivo replication. The nucleic acid can comprise any nucleotide sequence, which encodes any of the TCRs, polypeptides, or proteins, or functional portions or functional variants thereof described herein.

Furthermore, the invention provides a vector comprising a nucleic acid in accordance to the invention as described above. Desirably, the vector is an expression vector or a recombinant expression vector. The term "recombinant expression vector" refers in context of the present invention to a nucleic acid construct that allows for the expression of an mRNA, protein or polypeptide in a suitable host cell. The recombinant expression vector of the invention can be any suitable recombinant expression vector and can be used to transform or transfect any suitable host. Suitable vectors include those designed for propagation and expansion or for expression or both, such as plasmids and viruses. Examples of animal expression vectors include pEUK-Cl, pMAM, and pMAMneo. Preferably, the recombinant expression vector is a viral vector, e.g., a retroviral vector. The recombinant expression vector comprises regulatory sequences, such as transcription and translation initiation and termination codons, which are specific to the type of host cell (e.g., bacterium, fungus, plant, or animal), into which the vector is to be introduced and in which the expression of the nucleic acid of the invention may be performed. Furthermore, the vector of the invention may include one or more marker genes, which allow for selection of transformed or transfected hosts. The recombinant expression vector can comprise a native or normative promoter operably linked to the nucleotide sequence encoding the constructs of the invention, or to the nucleotide sequence, which is complementary to or which hybridizes to the nucleotide sequence encoding the constructs of the invention. The selections of promoters include, e.g., strong, weak, inducible, tissue-specific and developmental-specific promoters. The promoter can be a non-viral promoter or a viral promoter. The inventive recombinant expression vectors can be designed for either transient expression, for stable expression, or for both. Also, the recombinant expression vectors can be made for constitutive expression or for inducible expression.

The invention also pertains to a host cell comprising an antigen recognizing construct in accordance with the invention. Specifically, the host cell of the invention comprises a nucleic acid, or a vector as described herein above. The host cell can be a eukaryotic cell, e.g., plant, animal, fungi, or algae, or can be a prokaryotic cell, e.g., bacteria or protozoa. The host cell can be a cultured cell or a primary cell, i.e., isolated directly from an organism, e.g., a human. The host cell can be an adherent cell or a suspended cell, i.e., a cell that grows in suspension. For purposes of producing a recombinant TCR, polypeptide, or protein, the host cell is preferably a mammalian cell, e.g., Chinese Hamster Ovary (CHO) cell. Most preferably, the host cell is a human cell. While the host cell can be of any cell type, can originate from any type of tissue, and can be of any developmental stage, the host cell preferably is a peripheral blood leukocyte (PBL) or a peripheral blood mononuclear cell (PBMC). More preferably, the host cell is a T cell. The T cell can be any T cell, such as a cultured T cell, e.g., a primary T cell, or a T cell from a cultured T cell line, e.g., Jurkat, SupT1, etc., or a T cell obtained from a mammal, preferably a T cell or T cell precursor from a human patient. If obtained from a mammal, the T cell can be obtained from numerous sources, including but not limited to blood, bone marrow, lymph node, the thymus, or other tissues or fluids. T cells can also be enriched for or purified. Preferably, the T cell is a human T cell. More preferably, the T cell is a T cell isolated from a human. The T cell can be any type of T cell and can be of any developmental stage, including but not limited to, CD4-positive and/or CD8-positive, CD4-positive helper T cells, e.g., Th1 and Th2 cells, CD8-positive T cells (e.g., cytotoxic T cells), tumor infiltrating cells (TILs), memory T cells, naive T cells, and the like. Preferably, the T cell is a CD8-positive T cell or a CD4-positive T cell.

Preferably, the host cell of the invention is a lymphocyte, preferably, a T lymphocyte, such as a CD4-positive or CD8-positive T-cell. The host cell furthermore preferably is a tumor reactive T cell specific for COL6A3 expressing tumor cells.

One further aspect of the present invention relates to the herein disclosed antigen recognizing constructs, nucleic acids, vectors, pharmaceutical compositions and/or host cell for use in medicine. The use in medicine in one preferred embodiment includes the use in the diagnosis, prevention and/or treatment of a tumor disease, such as a malignant or benign tumor disease. The tumor disease is characterized by the expression of COL6A3, in a cancer or tumor cell of said tumor disease.

With respect to the above mentioned medical applications of the antigen recognizing constructs and other materials derived therefrom, the to be treated and/or diagnosed cancer can be any cancer, including any of acute lymphocytic cancer, acute myeloid leukemia, alveolar rhabdomyosarcoma, bone cancer, brain cancer, breast cancer, cancer of the anus, anal canal, or anorectum, cancer of the eye, cancer of the intrahepatic bile duct, cancer of the joints, cancer of the neck, gallbladder, or pleura, cancer of the nose, nasal cavity, or middle ear, cancer of the oral cavity, cancer of the vagina, cancer of the vulva, chronic lymphocytic leukemia, chronic myeloid cancer, colon cancer, esophageal cancer, cervical cancer, gastrointestinal carcinoid tumor, glioma, Hodgkin lymphoma, hypopharynx cancer, kidney cancer, larynx cancer, liver cancer, lung cancer, malignant mesothelioma, melanoma, multiple myeloma, nasopharynx cancer, non-Hodgkin lymphoma, cancer of the oropharynx, ovarian cancer, cancer of the penis, pancreatic cancer, peritoneum, omentum, and mesentery cancer, pharynx cancer, prostate cancer, rectal cancer, renal cancer, skin cancer, small intestine cancer, soft tissue cancer, stomach cancer, testicular cancer, thyroid cancer, cancer of the uterus, ureter cancer, and urinary bladder cancer. A preferred cancer is cancer is cancer of the uterine cervix, oropharynx, anus, anal canal, anorectum, vagina, vulva, or penis. A particularly preferred cancer is a COL6A3 positive cancer, including gastrointestinal and gastric cancer.

The constructs, TCRs, and nucleic acids of the invention are in particular for use in immune therapy, preferably, in adoptive T cell therapy. The administration of the compounds of the invention can, for example, involve the infusion of T cells of the invention into said patient. Preferably, such T cells are autologous T cells of the patient and *in vitro* transduced with a nucleic acid or antigen recognizing construct of the present invention.

WO 2016/011210 discloses engineered cells for adoptive therapy, including NK cells and T cells, and compositions containing the cells, and methods for their administration to subjects. The cells can contain genetically engineered antigen receptors that specifically bind to antigens, such as chimeric antigen receptors (CARs) and costimulatory receptors.

The object of the invention is also solved by a method of manufacturing a COL6A3 specific antigen recognizing construct expressing cell line, comprising
a. providing a suitable host cell,
b. providing a genetic construct comprising a coding sequence encoding the antigen recognizing construct according to any of claims 1 to 5,
c. introducing said genetic construct into said suitable host cell, and
d. expressing said genetic construct by said suitable host cell.

The method may further comprise a step of cell surface presentation of said antigen recognizing construct on said suitable host cell.

In other preferred embodiments, the genetic construct is an expression construct comprising a promoter sequence operably linked to said coding sequence.

Preferably, said antigen recognizing construct is of mammalian origin, preferably of human origin. The preferred suitable host cell for use in the method of the invention is a mammalian cell, such as a human cell, in particular a human T lymphocyte. T cells for use in the invention are described in detail herein above.

Also encompassed by the invention are embodiments, wherein said antigen recognizing construct is a modified TCR, wherein said modification is the addition of functional domains, such as a label or a therapeutically active substance. Furthermore, encompassed are TCR having alternative domains, such as an alternative membrane anchor domain instead of the endogenous transmembrane region.

Desirably, the transfection system for introducing the genetic construct into said suitable host cell is a retroviral vector system. Such systems are well known to the skilled artisan.

Also comprised by the present invention is in one embodiment the additional method step of isolation and purification of the antigen recognizing construct from the cell and, optionally, the reconstitution of the translated antigen recognizing construct-fragments in a T-cell.

In an alternative aspect of the invention a T-cell is provided obtained or obtainable by a method for the production of a T cell receptor (TCR), which is specific for tumorous cells and has high avidity as described herein above. Such a T cell is depending on the host cell used in the method of the invention, for example, a human or non-human T-cell, preferably a human TCR.

The inventive TCRs (including functional variants thereof), nucleic acids, recombinant expression vectors and host cells (including populations thereof), can be isolated and/or purified. The term "isolated" as used herein means having been removed from its natural environment. The term "purified" as used herein means having been increased in purity, wherein "purity" is a relative term, and not to be necessarily construed as absolute purity. For example, the purity can be at least about 50%, can be greater than 60%, 70%o, 80%, 90%, 95%, or can be 100%.

The inventive antigen recognizing constructs, TCRs, nucleic acids, recombinant expression vectors, host cells (including populations thereof), all of which are collectively referred to as "inventive TCR materials" hereinafter, can be formulated into a composition, such as a pharmaceutical composition. In this regard, the invention provides a pharmaceutical composition comprising any of the antigen recognizing constructs, TCRs, functional portions, functional variants, nucleic acids, expression vectors, host cells (including populations thereof) described herein, and a pharmaceutically acceptable carrier, excipient and/or stabilizer. The inventive pharmaceutical compositions containing any of the inventive TCR materials can comprise more than one inventive TCR material, e.g., a polypeptide and a nucleic acid, or two or more different TCRs (including functional portions and functional variants thereof). Alternatively, the pharmaceutical composition can comprise an inventive TCR material in combination with another pharmaceutically active agent(s) or drug(s), such as chemotherapeutic agents, e.g., asparaginase, busulfan, carboplatin, cisplatin, daunorubicin, doxorubicin, fluorouracil, gemcitabine, hydroxyurea, methotrexate, paclitaxel, rituximab, vinblastine, vincristine, etc. Preferably, the carrier is a pharmaceutically acceptable carrier. With respect to pharmaceutical compositions, the carrier can be any of those conventionally used for the particular inventive TCR material under consideration. Such pharmaceutically acceptable carriers are well-known to those skilled in the art and are readily available to the public. It is preferred that the pharmaceutically acceptable carrier be one, which has no detrimental side effects or toxicity under the conditions of use.

Thus, also provided is a pharmaceutical composition, comprising any of the herein described products of the invention and TCR materials of the invention, specifically any proteins, nucleic acids or host cells. In a preferred embodiment the pharmaceutical composition is for immune therapy, preferably adoptive cell therapy.

Preferably, the TCR material is administered by injection, e.g., intravenously. When the inventive TCR material is a host cell expressing the inventive TCR (or functional variant thereof), the pharmaceutically acceptable carrier for the cells for injection may include any isotonic carrier such as, for example, normal saline (about 0.90% w/v of NaCl in water, about 300 mOsm/L NaCl in water, or about 9.0 g NaCl per liter of water), NORMOSOL R electrolyte solution (Abbott, Chicago, IL), PLASMA-LYTE A (Baxter, Deerfield, IL), about 5% dextrose in water, or Ringer's lactate. In an embodiment, the pharmaceutically acceptable carrier is supplemented with human serum albumin.

For purposes of the invention, the amount or dose (e.g., numbers of cells when the inventive TCR material is one or more cells) of the inventive TCR material administered may be sufficient to affect, e.g., a therapeutic or prophylactic response, in the subject or animal over a reasonable time frame. For example, the dose of the inventive TCR material should be sufficient to bind to a cancer antigen, or detect, treat or prevent cancer in a period of from about 2 hours or longer, e.g., 12 to 24 or more hours, from the time of administration. In certain embodiments, the time period could be even longer. The dose will be determined by the efficacy of the particular inventive TCR material and the condition of the animal (e.g., human), as well as the body weight of the animal (e.g., human) to be treated.

It is contemplated that the inventive pharmaceutical compositions, TCRs (including functional variants thereof), polypeptides, proteins, nucleic acids, recombinant expression vectors, host cells, or populations of cells can be used in methods of treating or preventing cancer, or COL6A3-positive premalignancy. The inventive TCRs (and functional variants thereof) are believed to bind specifically to COL6A3 antigen, such that the TCR (or related inventive polypeptide or protein and functional variants thereof), when expressed by a cell, is able to mediate an immune response against a target cell expressing the COL6A3 antigens of the invention. In this regard, the invention provides a method of treating or preventing a condition, in particular cancer, in a mammal, comprising administering to the mammal any of the pharmaceutical compositions, antigen recognizing constructs, in particular TCRs (and functional variants thereof), polypeptides, or proteins described herein, any nucleic acid or recombinant expression vector comprising a nucleotide sequence encoding any of the TCRs (and functional variants thereof), polypeptides, proteins described herein, or any host cell or population of cells comprising a recombinant vector, which encodes any of the constructs of the invention (and functional variants thereof), polypeptides, or proteins described herein, in an amount effective to treat or prevent the condition in the mammal, wherein the condition is cancer, preferably COL6A3 positive cancer.

Examples of pharmaceutically acceptable carriers or diluents useful in the present invention include stabilizers such as SPGA, carbohydrates (e.g. sorbitol, mannitol, starch, sucrose, glucose, dextran), proteins such as albumin or casein, protein containing agents such as bovine serum or skimmed milk and buffers (e.g. phosphate buffer).

The terms "treat," and "prevent" as well as words stemming therefrom, as used herein, do not necessarily imply 100% or complete treatment or prevention. Rather, there are varying degrees of treatment or prevention of which one of ordinary skill in the art recognizes as having a potential benefit or therapeutic effect. In this respect, the methods described herein can provide any amount of any level of treatment or prevention of a condition in a mammal. Furthermore, the treatment or prevention provided by the method described herein can include treatment or prevention of one or more conditions or symptoms of the condition, e.g., cancer, being treated or prevented. For example, treatment or prevention can include promoting the regression of a tumor. Also, for purposes herein, "prevention" can encompass delaying the onset of the condition, or a symptom or condition thereof.

Disclosed but not covered by the claimed invention is a method of treating cancer comprising administering a TCR, a nucleic acid, or a host cell of the present description in combination with at least one chemotherapeutic agent and/or radiation therapy.

Also provided is an in-vitro method of treating cancer or COL6A3-positive premalignancy wherein said cancer is selected from a cancer where COL6A3 is overexpressed, mutated, and/or a COL6A3-derived tumor-associated antigen is presented, the method, comprising:
a) transforming a cell obtained from a subject in need of a cancer treatment with at least one vector encoding an antigen recognizing construct of the present invention to produce a transformed cell;
c) expanding the transformed cell to produce a plurality of transformed cells;

Also disclosed but not being covered by the claimed invention is a method of treating cancer in a subject in need thereof, comprising:
a) isolating a cell from a healthy donor;
b) transforming the cell with a vector encoding an antigen recognizing construct of the present invention to produce a transformed cell;
c) expanding the transformed cell to produce a plurality of transformed cells; and
d) administering the plurality of transformed cells to said subject.

Also provided is an in vitro method of detecting cancer in a biological sample comprising:
a) contacting the biological sample with an antigen recognizing construct of the present description;
b) detecting binding of the antigen recognizing construct to the biological sample; wherein said cancer is selected from a cancer where COL6A3 is overexpressed, mutated, and/or a COL6A3-derived tumor-associated antigen is presented.

In some embodiments, the method of detecting cancer is carried out in vitro, in vivo or in situ.

Also disclosed but not being covered by the claimed invention is a method of detecting the presence of a condition in a mammal. The method comprises (i) contacting a sample comprising one or more cells from the mammal with any of the inventive TCRs (and functional variants thereof), polypeptides, proteins, nucleic acids, recombinant expression vectors, host cells, populations of cells, antibodies, or antigen binding portions thereof, or pharmaceutical compositions described herein, thereby forming a complex, and (ii) detecting the complex, wherein detection of the complex is indicative of the presence of the condition in the mammal, wherein the condition is cancer, such as a COL6A3-positive malignancy.

With respect to the method of detecting a condition in a mammal, the sample of cells can be a sample comprising whole cells, lysates thereof, or a fraction of the whole cell lysates, e.g., a nuclear or cytoplasmic fraction, a whole protein fraction, or a nucleic acid fraction.

For purposes of the inventive detecting method, the contacting is in vitro.

Also, detection of the complex can occur through any number of ways known in the art. For instance, the inventive antigen recognizing constructs (and functional variants thereof), nucleic acids, recombinant expression vectors, host cells, populations of cells, or TCRs, or antigen binding portions thereof, described herein, can be labeled with a detectable label such as, for instance, a radioisotope, a fluorophore (e.g., fluorescein isothiocyanate (FITC), phycoerythrin (PE)), an enzyme (e.g., alkaline phosphatase, horseradish peroxidase), and element particles (e.g., gold particles).

For purposes of the inventive methods, wherein host cells or populations of cells are administered, the cells can be cells that are allogeneic or autologous to the mammal. Preferably, the cells are autologous to the mammal.

With respect to the above mentioned medical applications of the TCR material of the invention, the to be treated and/or diagnosed cancer can be any cancer, including any of acute lymphocytic cancer, acute myeloid leukemia, alveolar rhabdomyosarcoma, bone cancer, brain cancer, breast cancer, cancer of the anus, anal canal, or anorectum, cancer of the eye, cancer of the intrahepatic bile duct, cancer of the joints, cancer of the neck, gallbladder, or pleura, cancer of the nose, nasal cavity, or middle ear, cancer of the oral cavity, cancer of the vagina, cancer of the vulva, chronic lymphocytic leukemia, chronic myeloid cancer, colon cancer, esophageal cancer, cervical cancer, gastrointestinal carcinoid tumor, glioma, Hodgkin lymphoma, hypopharynx cancer, kidney cancer, larynx cancer, liver cancer, lung cancer, malignant mesothelioma, melanoma, multiple myeloma, nasopharynx cancer, non-Hodgkin lymphoma, cancer of the oropharynx, ovarian cancer, cancer of the penis, pancreatic cancer, peritoneum, omentum, and mesentery cancer, pharynx cancer, prostate cancer, rectal cancer, renal cancer, skin cancer, small intestine cancer, soft tissue cancer, stomach cancer, testicular cancer, thyroid cancer, cancer of the uterus, ureter cancer, and urinary bladder cancer. A preferred cancer is cancer of the uterine cervix, oropharynx, anus, anal canal, anorectum, vagina, vulva, or penis. A particularly preferred cancer is a COL6A3 positive cancer, such as gastrointestinal or gastric cancer.

In general, the invention provides a method for treating a subject suffering from a tumor or tumor disease comprising the administration of the antigen recognizing constructs, nucleic acids, vectors, pharmaceutical compositions and/or host cell as disclosed by the present invention. Preferably the subject is a subject in need of such a treatment. The subject in preferred embodiments is a mammalian subject, preferably a human patient, suffering from a tumor or tumor disease, which is COL6A3-positive.

The present invention will now be further described in the following examples with reference to the accompanying figures and sequences, nevertheless, without being limited thereto. In the Figures and Sequences:

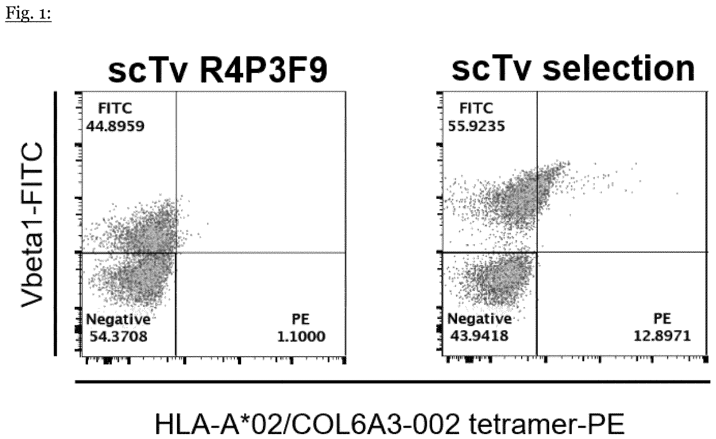

Figure 1 shows the conversion of a TCR into stabilized Vα/Vβ single-chain TCR (scTv) via yeast surface display. ScTv molecules displayed on the surface of transformed Saccharomyces cerevisiae EBY100 were stained with FITC-labeled anti-Vbetal antibody and PE-labeled HLA-A^{∗}02/COL6A3-002 tetramer. The unmodified scTv R4P3F9 (left panel, SEQ ID NO: 22) is compared to an scTv clone bearing single point mutations to stabilize the scTv scaffold (right panel), which was derived from the selection of a random mutation scTv library.

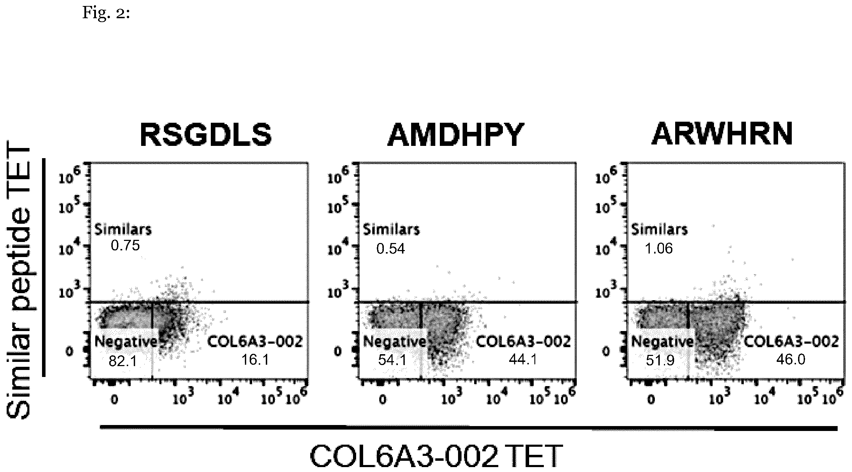

Figure 2 shows scTv affinity maturation via yeast surface display. Stabilized scTv molecules with and without affinity maturated CDR1 beta were stained with HLA-A^{∗}02 tetramers containing COL6A3-002 (SEQ ID NO: 1) and counterstained with a mix of HLA-A^{∗}02 tetramers containing 9 peptides (SEQ ID NO: 28 to 36) with high sequence similarity to COL6A3-002. Stabilized scTv (SEQ ID NO 27) with non-maturated beta chain CDR1 sequence RSGDLS (SEQ ID NO: 13) is compared with scTv clones bearing the affinity maturated beta chain CDR1 sequences AMDHPY (SEQ ID NO: 40) and ARWHRN (SEQ ID NO: 39).

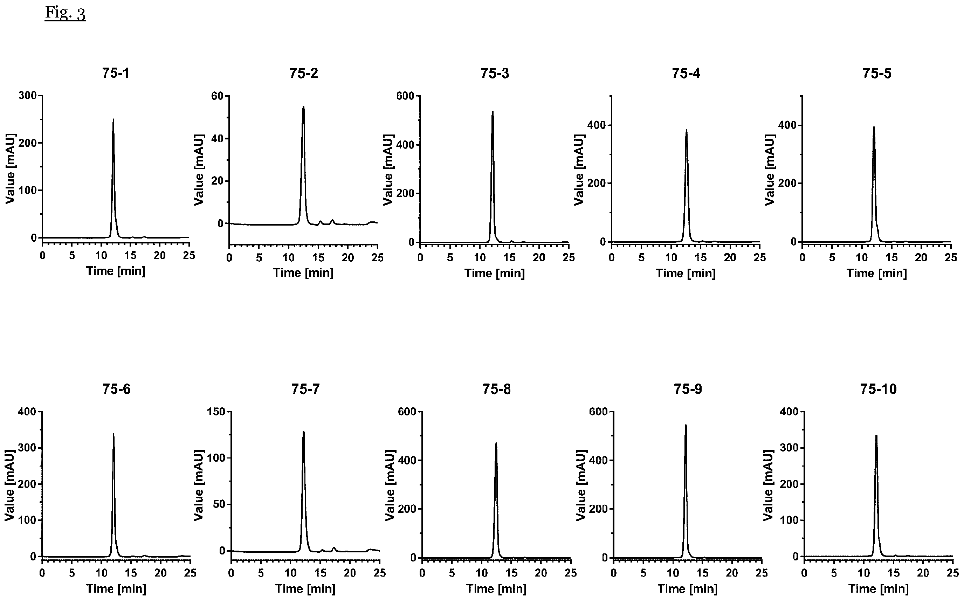

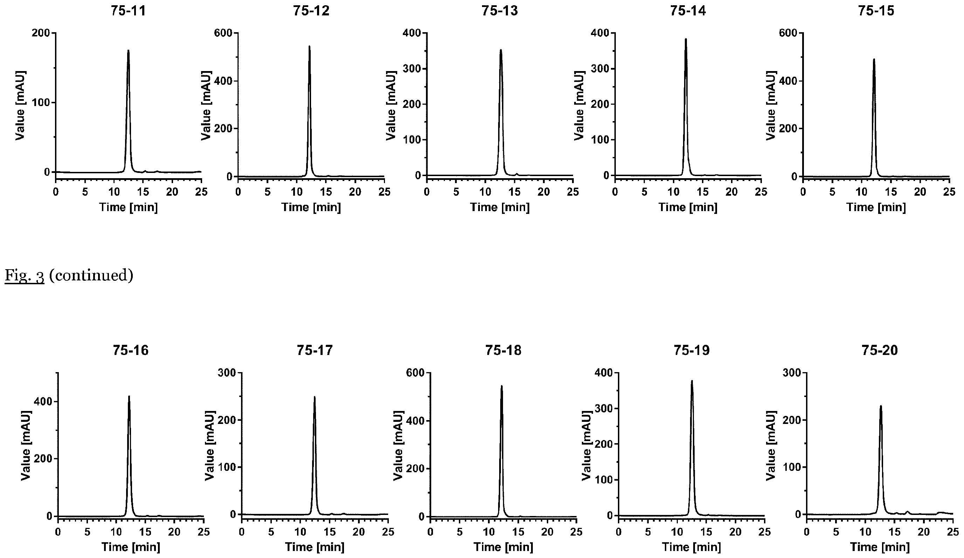

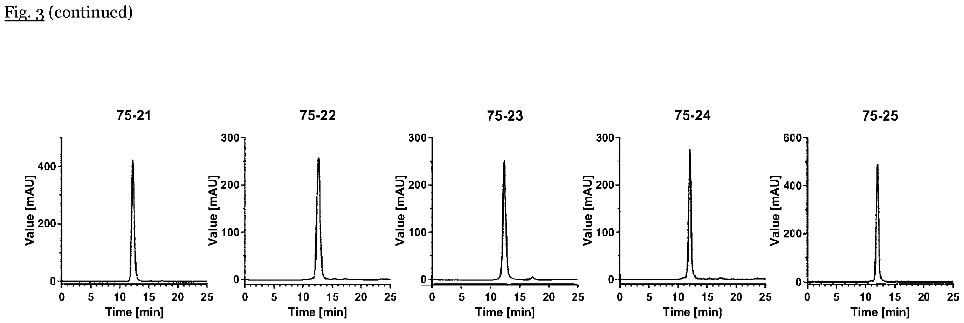

Figure 3 shows size exclusion chromatography elution profiles of anti-CD3 Fab-scTv R4P3F9S fusion variants 75-1 to 75-25.

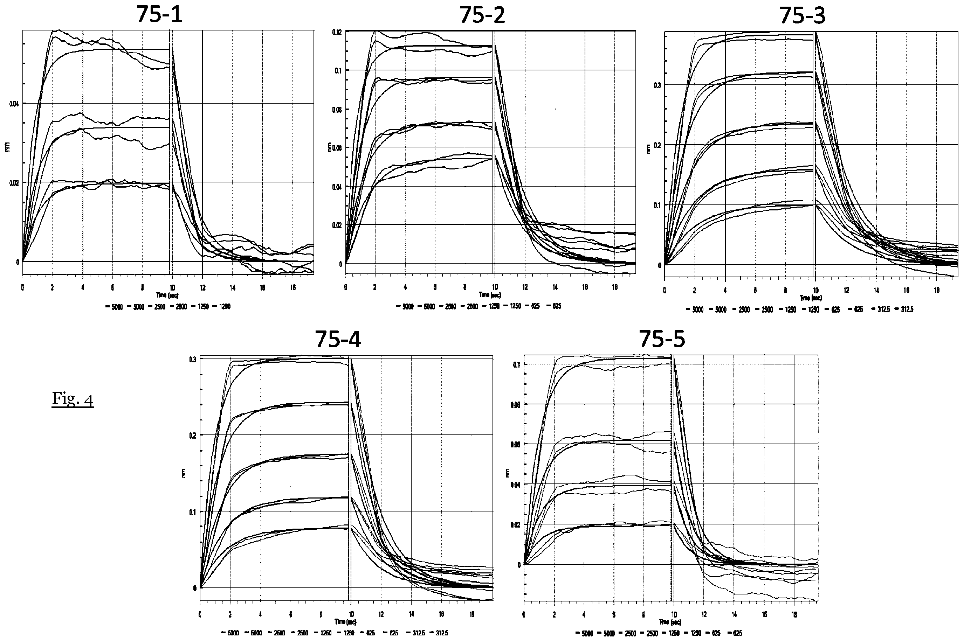

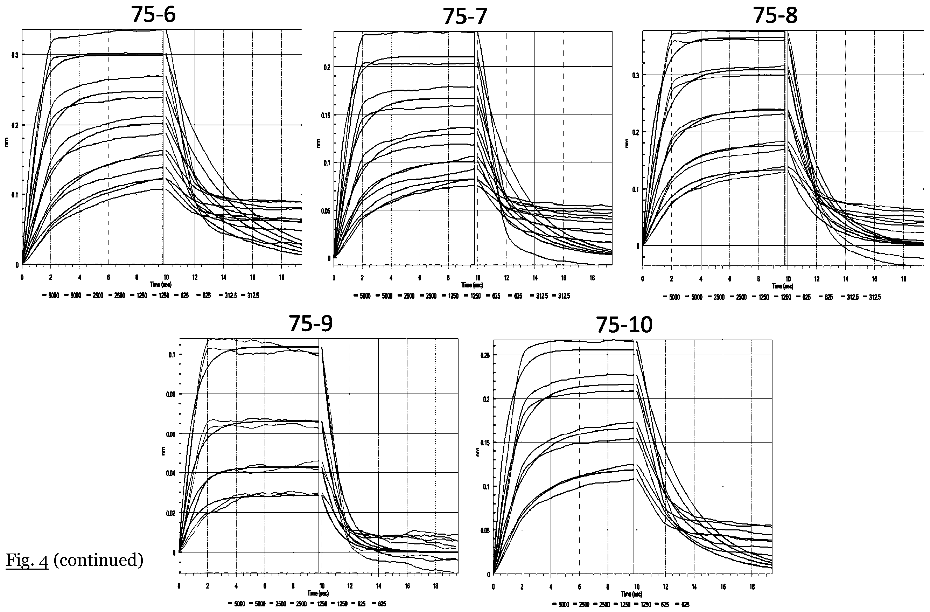

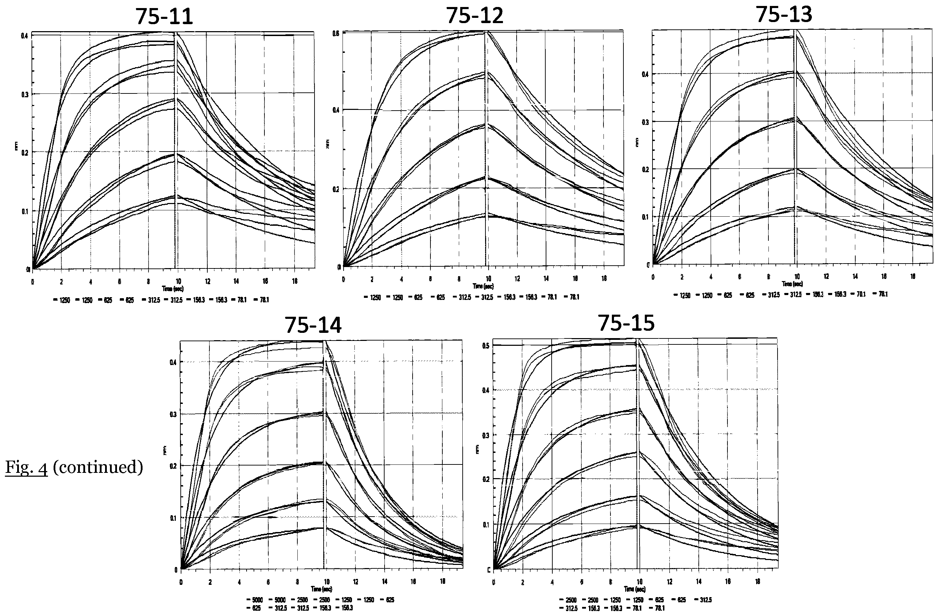

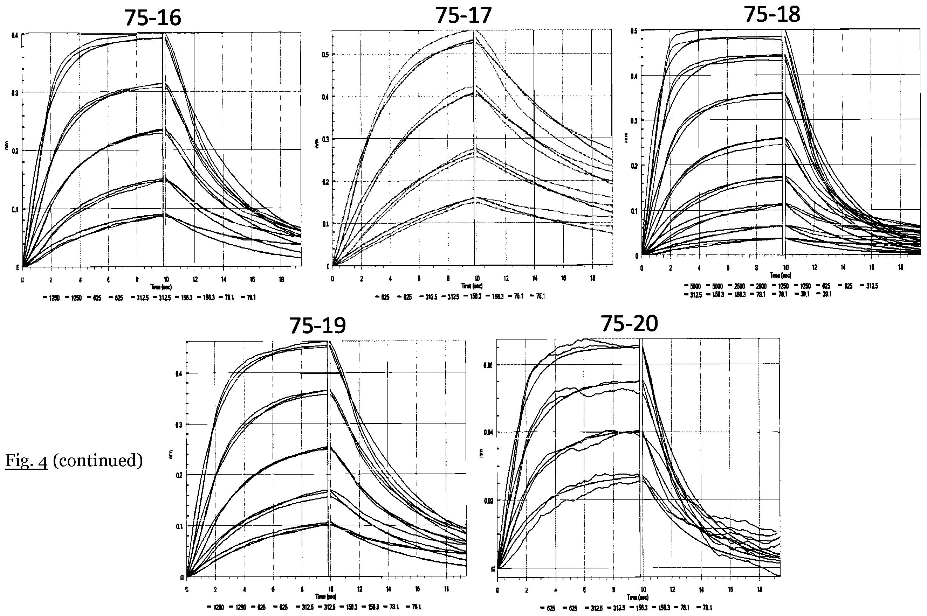

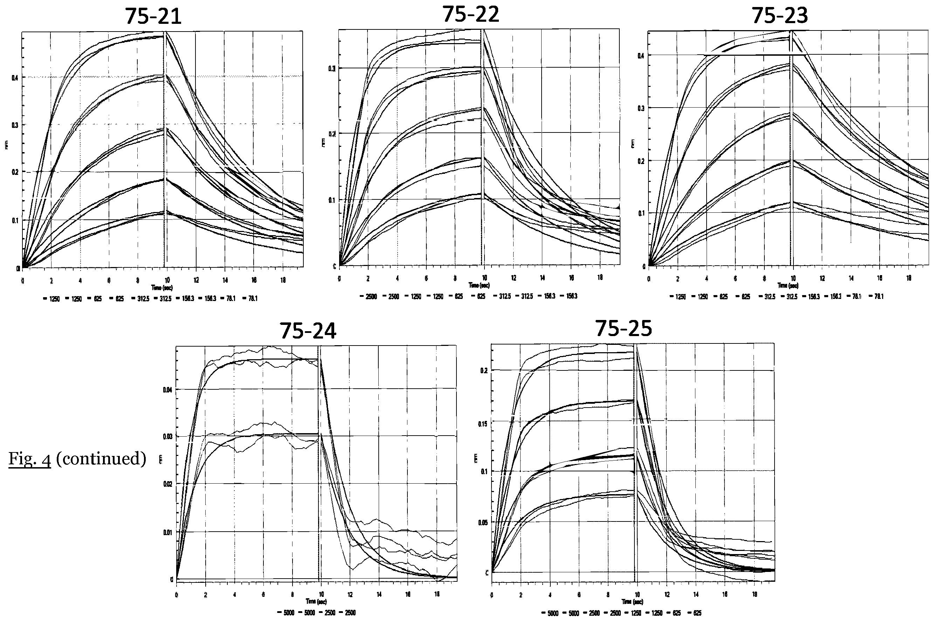

Figure 4 shows the HLA-A^{∗}02/COL6A3-002 binding kinetics of anti-CD3 Fab-scTv R4P3F9S fusion variants 75-1 to 75-25 as measured by biolayer interferometry. Analyzed concentrations of HLA-A^{∗}02/COL6A3-002 are indicated.

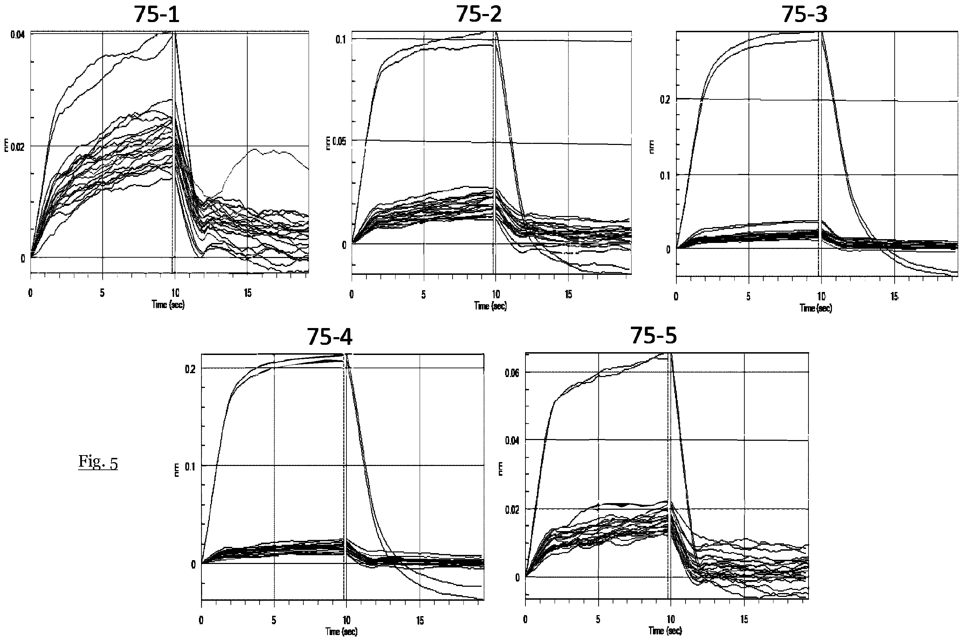

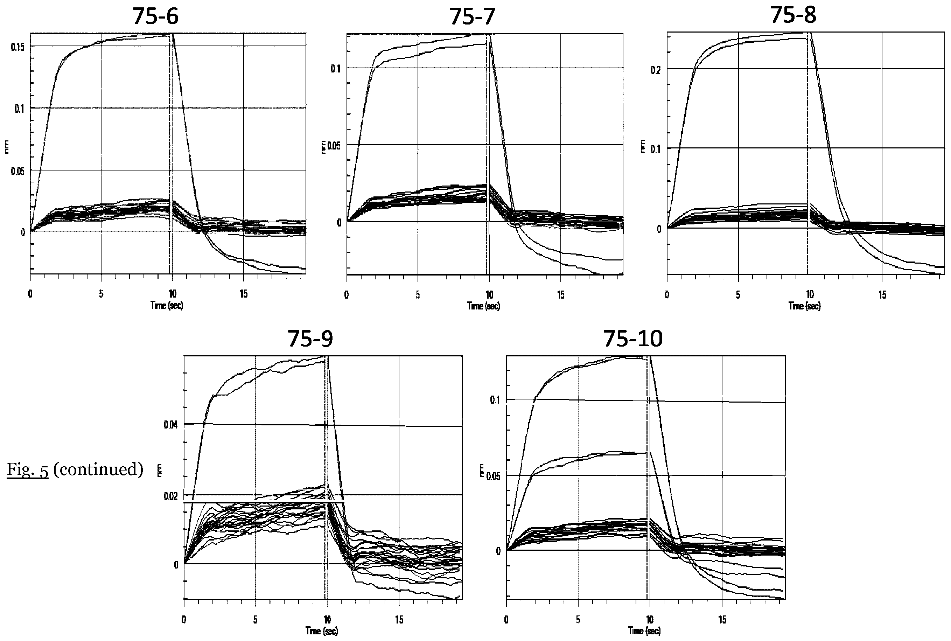

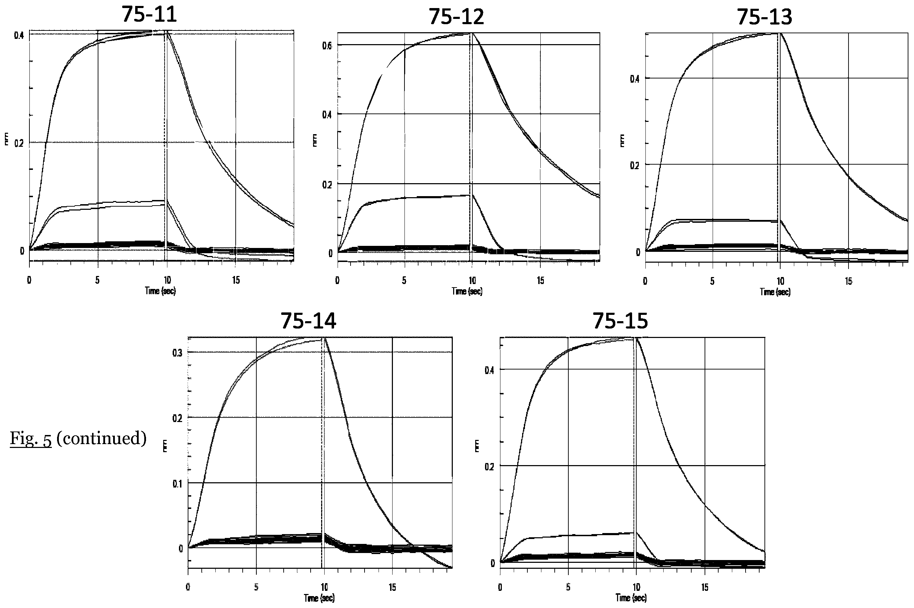

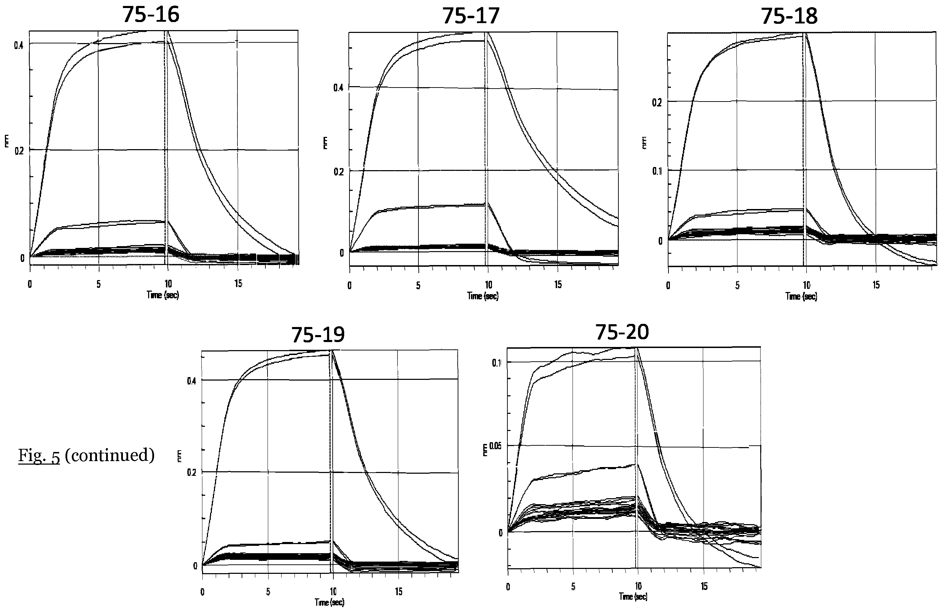

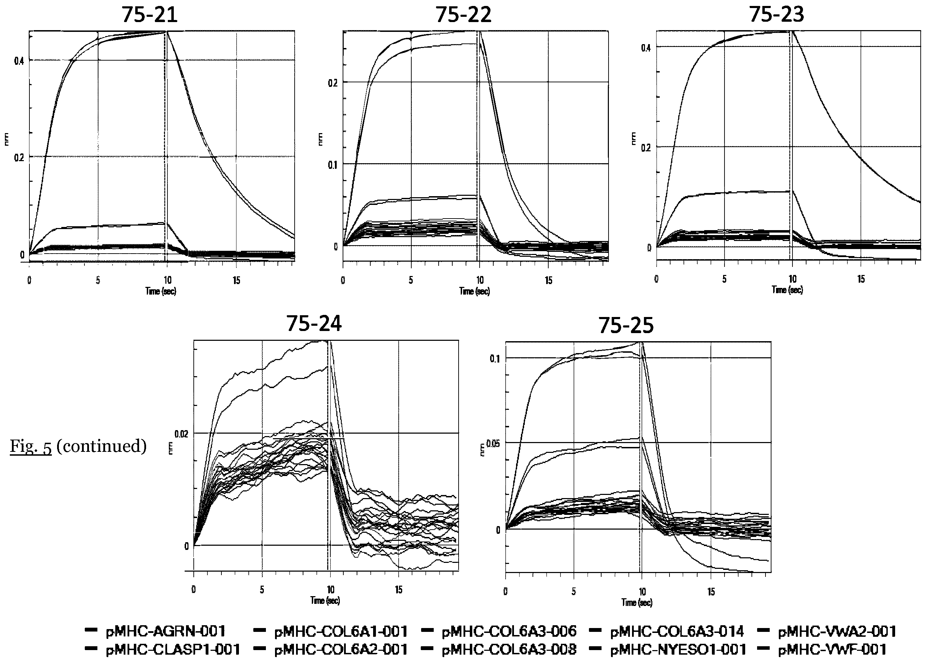

Figure 5 shows binding analysis of anti-CD3 Fab-scTv R4P3F9S fusion variants 75-1 to 75-25 as measured via biolayer interferometry (BLI). 1 µM of HLA-A^{∗}02 in complex with the indicated similar peptides was analyzed.

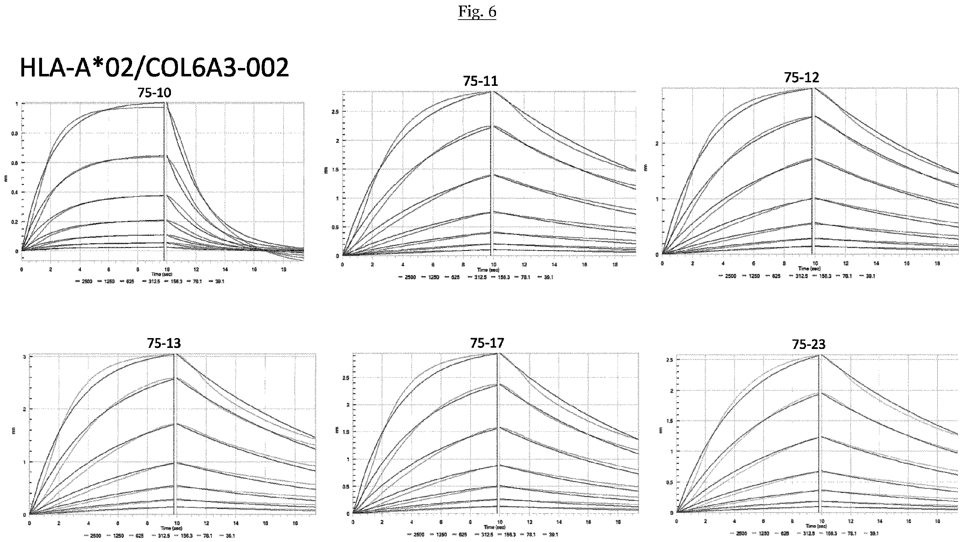

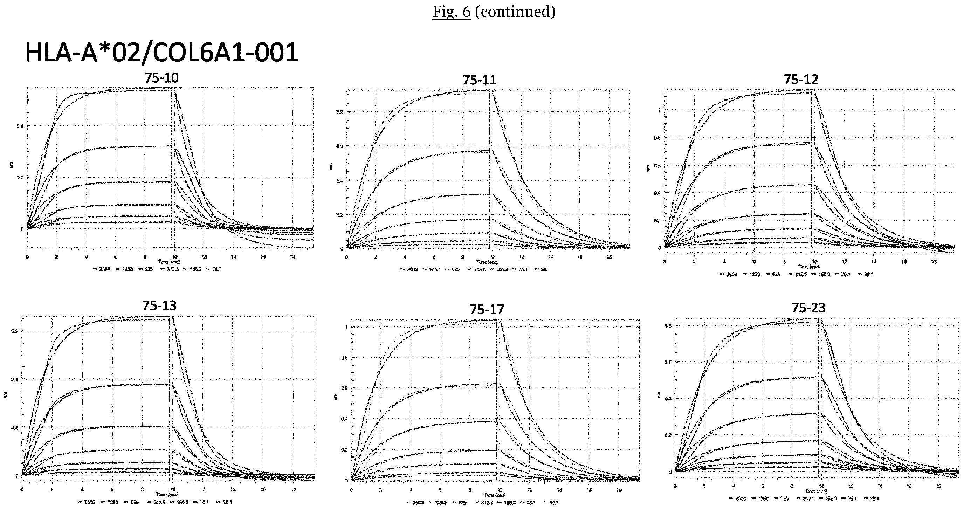

Figure 6 shows a comparison of HLA-A^{∗}02/COL6A3-002 and HLA-A^{∗}02/COL6A1-001 (SEQ ID NO: 30) binding kinetics of different anti-CD3 Fab-scTv R4P3F9S fusion variants. Analyzed concentrations of Fab-scTv molecules are indicated.

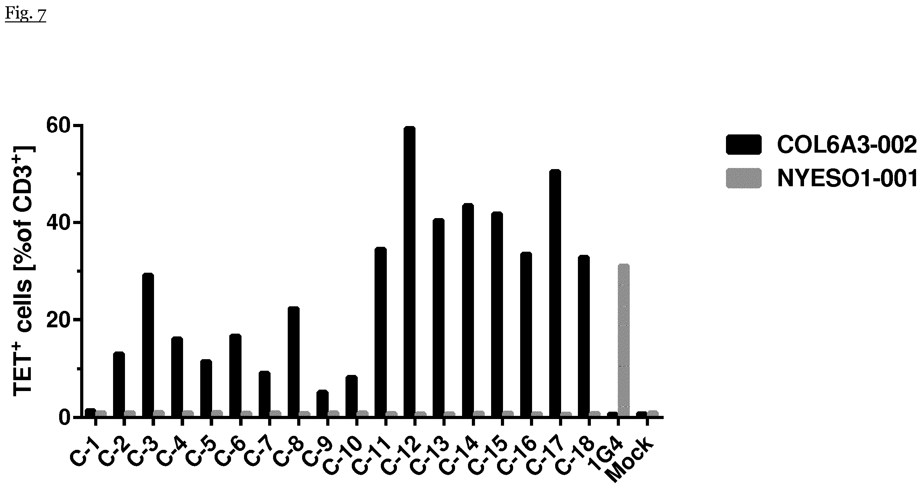

Figure 7 shows staining of maturated R4P3F9 TCR variant expressing human CD8⁺ T cells with PE-labeled HLA-A^{∗}02/COL6A3-002 tetramers. For control purpose, no TCR (Mock) or the 1G4 TCR specific for NYESO1-001 was expressed and staining with PE-labeled HLA-A^{∗}02/NYESO1-001 tetramers was used.

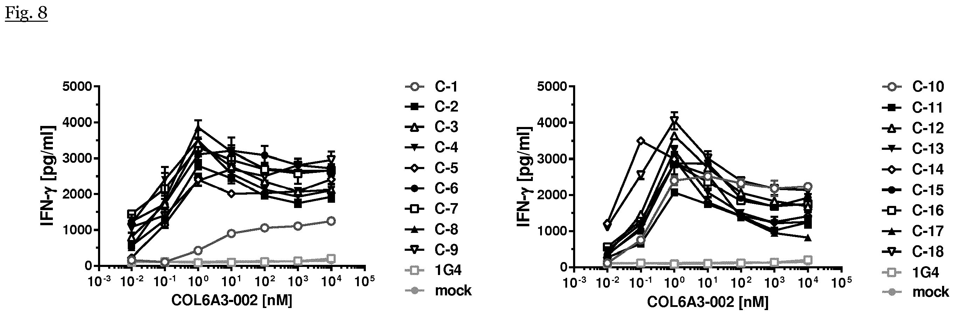

Figure 8 shows IFN-gamma release of maturated R4P3F9 TCR variant expressing human CD8⁺ T cells in response to COL6A3-002. For control purpose, no TCR (mock) or the 1G4 TCR specific for NYESO1-001 was expressed. IFN-gamma release was determined by ELISA after co-culture of electroporated CD8⁺ T cells with T2 cells loaded with a serial dilution of COL6A3-002.

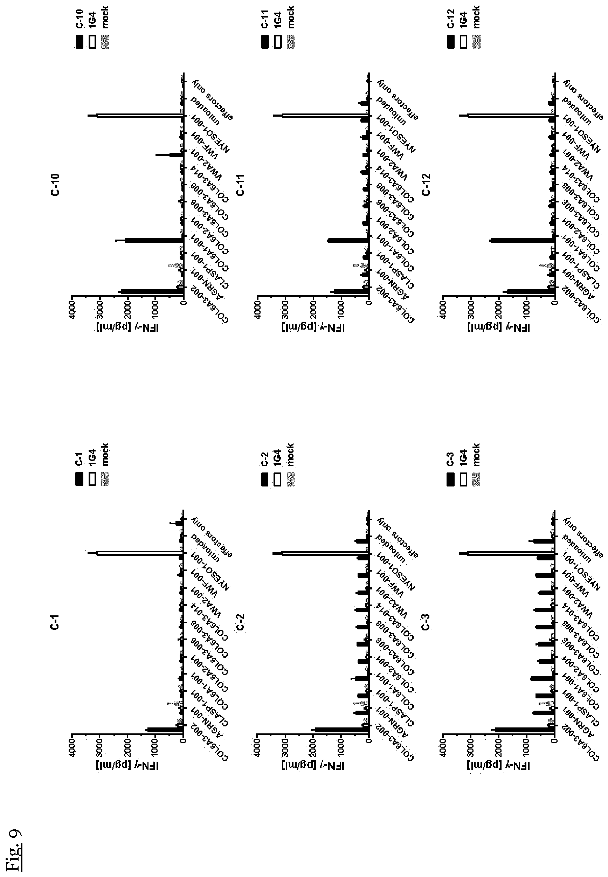

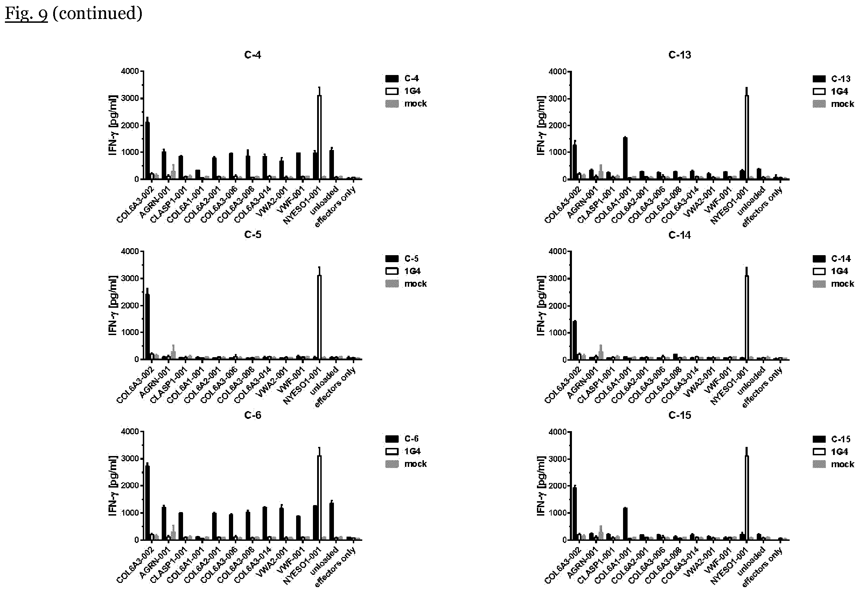

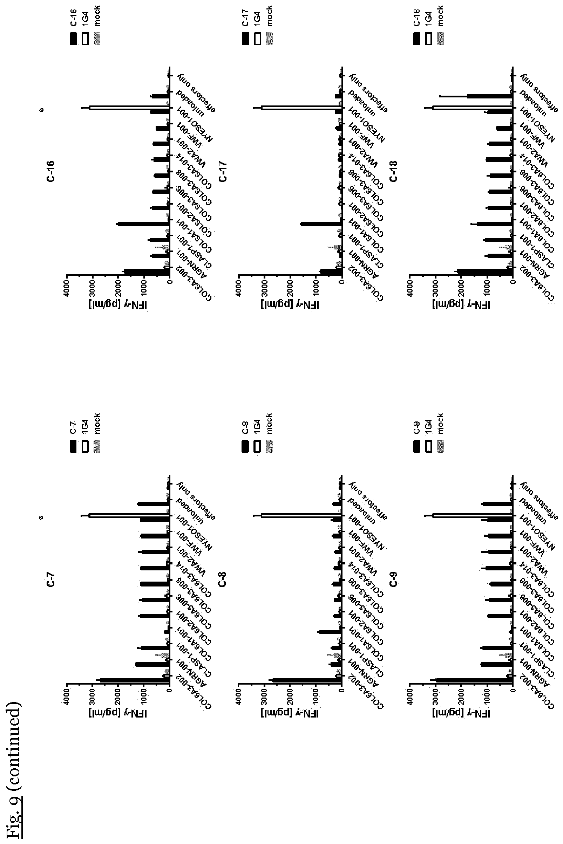

Figure 9 shows IFN-gamma release of maturated R4P3F9 TCR variant expressing human CD8⁺ T cells in response to COL6A3-002 and different similar peptides. For control purpose, no TCR (mock) or the 1G4 TCR specific for NYESO1-001 was expressed. IFN-gamma release was determined by ELISA after co-culture of electroporated CD8⁺ T cells with T2 cells loaded with 10 µM of COL6A3-002 or similar peptides.

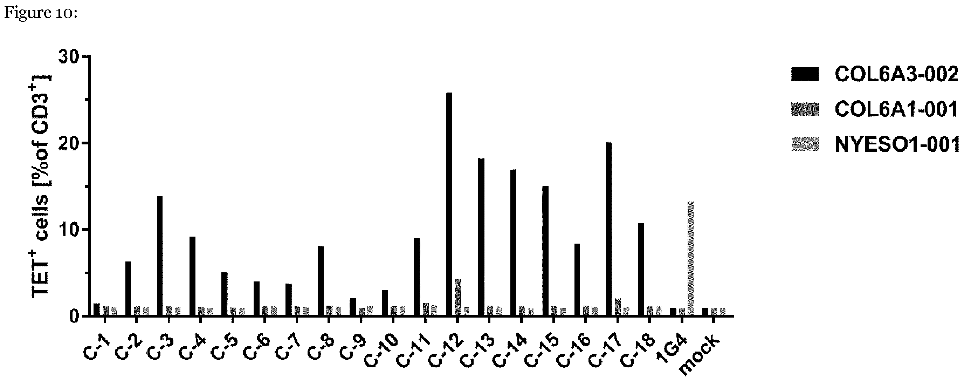

Figure 10 shows staining of maturated R4P3F9 TCR variant expressing human CD8⁺ T cells with PE-labeled peptide-HLA-A^{∗}02 tetramers. For control purpose, no TCR (Mock) or the 1G4 TCR specific for NYESO1-001 was expressed and staining with PE-labeled HLA-A^{∗}02/NYESO1-001 tetramers was used.

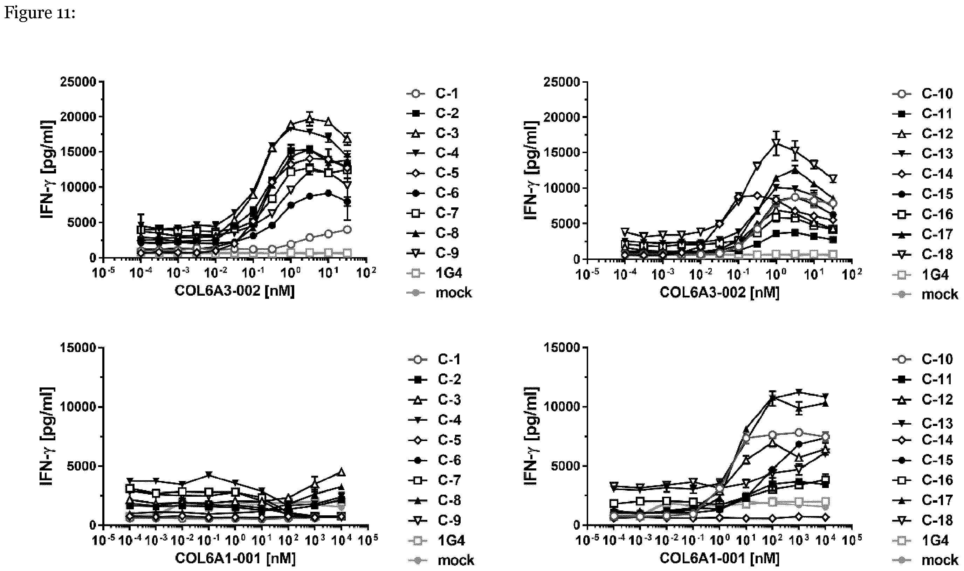

Figure 11 shows IFN-gamma release of maturated R4P3F9 TCR variant expressing human CD8⁺ T cells in response to COL6A3-002 or COL6A1-001. For control purpose, no TCR (mock) or the 1G4 TCR specific for NYESO1-001 was expressed. IFN-gamma release was determined by ELISA after co-culture of electroporated CD8⁺ T cells with T2 cells loaded with a serial dilution of COL6A3-002 or COL6A1-001.

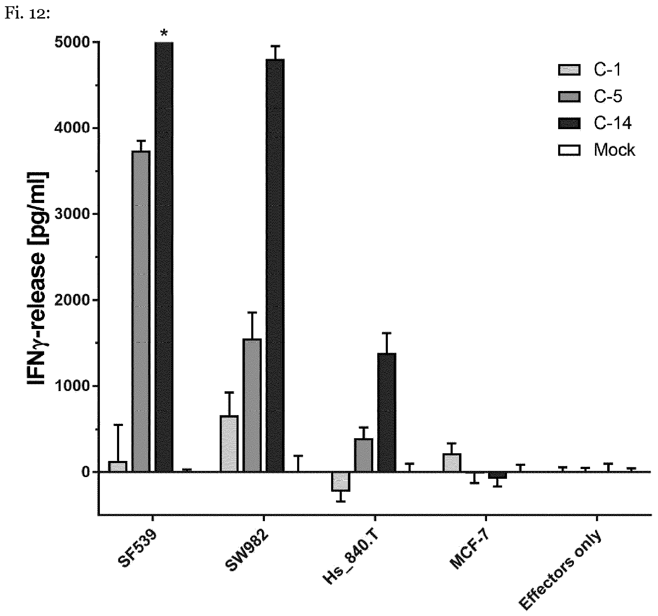

Figure 12 shows IFN-gamma release of primary human CD8⁺ T cells expressing R4P3F9 TCR variants upon co-culture with different tumor cell lines. SF539, SW982 and Hs840.T cells present the target peptide at different levels. MCF-7 cells do not present the target peptide. As controls, effector cells without exogenous TCR were analyzed along with cells with TCRs of interest. IFN-gamma release was determined by ELISA. ^{∗} marks a data point that is out of scale.

**Table 1: Peptide sequences of the invention (positions are according to IMGT numbering:**

| (François Ehrenmann, Patrice Duroux, Chantal Ginestoux; Protein displays: human (Homo sapiens) TRAV; IMGT Repertoire. IMGT^{®}, the international ImMunoGenetics information system^{®} http://www.imgt.org.; Created: 16/03/2011. Version: 03/06/2016; François Ehrenmann, Patrice Duroux, Chantal Ginestoux; Protein displays: human (Homo sapiens) TRBV; IMGT Repertoire. IMGT^{®}, the international ImMunoGenetics information system^{®} http://www.imgt.org.; Created: 16/03/2011. Version: 03/06/2016.) | | | |
|---|---|---|---|
| SEQ ID NO: | Name | Descriptio n | Sequence |
| 1 | COL6A3-002 | | FLLDGSANV |
| 2 | R4P3F9 alpha | R4P3F9 TCR alpha chain - full length | MKSLRVLLVILWLQLSWVWSQQKEVEQNSGPLSVPEGAIA SLNCTYSDRGSQSFFWYRQYSGKSPELIMFIYSNGDKEDG RFTAQLNKASQYVSLLIRDSQPSDSATYLCAAYSGAGSYQ LTFGKGTKLSVIPNIQNPDPAVYQLRDSKSSDKSVCLFTD FDSQTNVSQSKDSDVYITDKTVLDMRSMDFKSNSAVAWSN KSDFACANAFNNSIIPEDTFFPSPESSCDVKLVEKSFETD TNLNFQNLSVIGFRILLLKVAGFNLLMTLRLWSS |
| 3 | R4P3F9 alpha leader | R4P3F9 TCR alpha chain - leader peptide | MKSLRVLLVILWLQLSWVWSQ |
| 4 | R4P3F9 alpha variable | R4P3F9 TCR alpha chain - variable domain | QKEVEQNSGPLSVPEGAIASLNCTYSDRGSQSFFWYRQYS GKSPELIMFIYSNGDKEDGRFTAQLNKASQYVSLLIRDSQ PSDSATYLCAAYSGAGSYQLTFGKGTKLSVIP |
| 5 | R4P3F9 CDRal | R4P3F9 TCR alpha chain - CDR1 | DRGSQS |
| 6 | R4P3F9 CDRa2 | R4P3F9 TCR alpha chain - CDR2 | IYSNGD |
| 7 | R4P3F9 CDRa3 | R4P3F9 TCR alpha chain - CDR3 | CAAYSGAGSYQLT |
| 8 | R4P3F9 - alpha constant | R4P3F9 TCR alpha chain - constant domain | NIQNPDPAVYQLRDSKSSDKSVCLFTDFDSQTNVSQSKDS DVYITDKTVLDMRSMDFKSNSAVAWSNKSDFACANAFNNS IIPEDTFFPSPESSCDVKLVEKSFETDTNLNFQNLSVIGF RILLLKVAGFNLLMTLRLWSS |
| 9 | R4P3F9 - alpha constant start | R4P3F9 TCR alpha chain - constant domain start | NIQN |
| 10 | R4P3F9 beta | R4P3F9 TCR beta chain - full length | MGFRLLCCVAFCLLGAGPVDSGVTQTPKHLITATGQRVTL RCSPRSGDLSVYWYQQSLDQGLQFLIQYYNGEERAKGNIL ERFSAQQFPDLHSELNLSSLELGDSALYFCASSVESSYGY TFGSGTRLTVVEDLNKVFPPEVAVFEPSEAEISHTQKATL VCLATGFFPDHVELSWWVNGKEVHSGVSTDPQPLKEQPAL NDSRYCLSSRLRVSATFWQNPRNHFRCQVQFYGLSENDEW TQDRAKPVTQIVSAEAWGRADCGFTSVSYQQGVLSATILY EILLGKATLYAVLVSALVLMAMVKRKDF |
| 11 | R4P3F9 beta leader | R4P3F9 TCR beta chain - leader peptide | MGFRLLCCVAFCLLGAGPV |
| 12 | R4P3F9 beta variable | R4P3F9 TCR beta chain - variable domain | DSGVTQTPKHLITATGQRVTLRCSPRSGDLSVYWYQQSLD QGLQFLIQYYNGEERAKGNILERFSAQQFPDLHSELNLSS LELGDSALYFCASSVESSYGYTFGSGTRLTVV |
| 13 | R4P3F9 CDRbl | R4P3F9 TCR beta chain - CDR1 | RSGDLS |
| 14 | R4P3F9 CDRb2 | R4P3F9 TCR beta chain - CDR2 | YYNGEE |
| 15 | R4P3F9 CDRb3 | R4P3F9 TCR beta chain - CDR3 | CASSVESSYGYT |
| 16 | R4P3F9 beta constant | R4P3F9 TCR beta chain - constant domain | EDLNKVFPPEVAVFEPSEAEISHTQKATLVCLATGFFPDH VELSWWVNGKEVHSGVSTDPQPLKEQPALNDSRYCLSSRL RVSATFWQNPRNHFRCQVQFYGLSENDEWTQDRAKPVTQI VSAEAWGRADCGFTSVSYQQGVLSATILYEILLGKATLYA VLVSALVLMAMVKRKDF |
| 17 | R4P3F9 beta constant start 1 | R4P3F9 TCR beta chain - constant domain start 1 | EDLNK |
| 18 | R4P3F9 beta constant start 2 | R4P3F9 TCR beta chain - constant domain start 2 | EDLKN |
| 19 | Aga2p-R4P3F9 | Aga2p fusion protein with scTv R4P3F9 and tags | MQLLRCFSIFSVIASVLAQELTTICEQIPSPTLESTPYSL STTTILANGKAMQGVFEYYKSVTFVSNCGSHPSTTSKGSP INTQYVFGGGGSDYKDDDDKGGGASQKEVEQNSGPLSVPE GAIASLNCTYSDRGSQSFFWYRQYSGKSPELIMSIYSNGD KEDGRFTAQLNKASQYVSLLIRDSQPSDSATYLCAAYSGA GSYQLTFGKGTKLSVIPNIQNGGGGSGGGGSGGGGSGGGG SGVTQTPKHLITATGQRVTLRCSPRSGDLSVYWYQQSLDQ GLQFLIQYYNGEERAKGNILERFSAQQFPDLHSELNLSSL ELGDSALYFCASSVESSYGYTFGSGTRLTVVEDLNKAAAG GSGGEQKLISEEDL MQLLRCFSIFSVIASVLAQELTTICEQIPSPTLESTPYSL STTTILANGKAMQGVFEYYKSVTFVSNCGSHPSTTSKGSP INTQYVF |
| 20 | Aga2p | Leader sequence and Aga2p | MQLLRCFSIFSVIASVLAQELTTICEQIPSPTLESTPYSL STTTILANGKAMQGVFEYYKSVTFVSNCGSHPSTTSKGSP INTQYVF |
| 21 | FLAG tag | FLAG tag plus linkers | GGGGSDYKDDDDKGGGAS |
| 22 | scTv R4P3F9 | Single chain variable domains of R4P3F9 with linker; aF55S in alpha variable domain | QKEVEQNSGPLSVPEGAIASLNCTYSDRGSQSFFWYRQYS GKSPELIMSIYSNGDKEDGRFTAQLNKASQYVSLLIRDSQ PSDSATYLCAAYSGAGSYQLTFGKGTKLSVIPNIQNGGGG SGGGGSGGGGSGGGGSGVTQTPKHLITATGQRVTLRCSPR SGDLSVYWYQQSLDQGLQFLIQYYNGEERAKGNILERFSA QQFPDLHSELNLSSLELGDSALYFCASSVESSYGYTFGSG TRLTVVEDLNK |
| 23 | Myc tag | Linker and Myc tag | AAAGGSGGEQKLISEEDL |
| 24 | scTv R4P3F9-bQ43K | scTv R4P3F9 with stabilizin g mutation bQ43K in beta variable domain | QKEVEQNSGPLSVPEGAIASLNCTYSDRGSQSFFWYRQYS GKSPELIMSIYSNGDKEDGRFTAQLNKASQYVSLLIRDSQ PSDSATYLCAAYSGAGSYQLTFGKGTKLSVIPNIQNGGGG SGGGGSGGGGSGGGGSGVTQTPKHLITATGQRVTLRCSPR SGDLSVYWYKQSLDQGLQFLIQYYNGEERAKGNILERFSA QQFPDLHSELNLSSLELGDSALYFCASSVESSYGYTFGSG TRLTVVEDLNK QKEVEQNSGPLSVPEGAIASLNCTYSDRGSQSFFWYRQYS GKSPELIMSIYSNGDKEDGRFTAQLNKASQYVSLLIRDSQ PSDSATYLCAAYSGAGSYQLTFGKGTKLSVIPNIQNGGGG SGGGGSGGGGSGGGGSGVTQTPKHLITATGQRVTLRCSPR SGDLSVYWYQQSLDQGLQFLIQYYNGEERAKGNISERFSA QQFPDLHSELNLSSLELGDSALYFCASSVESSYGYTFGSG TRLTVVEDLNK |
| 25 | scTv R4P3F9-bL72S | scTv R4P3F9 with stabilizin g mutation bL72S in beta variable domain | QKEVEQNSGPLSVPEGAIASLNCTYSDRGSQSFFWYRQYS GKSPELIMSIYSNGDKEDGRFTAQLNKASQYVSLLIRDSQ PSDSATYLCAAYSGAGSYQLTFGKGTKLSVIPNIQNGGGG SGGGGSGGGGSGGGGSGVTQTPKHLITATGQRVTLRCSPR SGDLSVYWYQQSLDQGLQFLIQYYNGEERAKGNISERFSA QQFPDLHSELNLSSLELGDSALYFCASSVESSYGYTFGSG TRLTVVEDLNK |
| 26 | CDRal mutant1 | aG29R mutation | DRRSQS |
| 27 | scTv R4P3F9S | Stabilized version of scTv R4P3F9 | QKEVEQNSGPLSVPEGAIASLNCTYSDRRSQSFFWYRQYS GKSPELIMSIYSNGDKEDGRFTAQLNKASQYVSLLIRDSQ PSDSATYLCAAYSGAGSYQLTFGKGTKLSVIPGGGGSGGG GSGGGGSGGGGSGGGGSGVTQTPKHLITATGQRVTLRCSP RSGDLSVYWYKQSLDQGLQFLIQYYNGEERAKGNISERFS AQQFPDLHSELNLSSLELGDSALYFCASSVESSYGYTFGS GTRLTVV |
| 28 | AGRN-001 | Similar peptides | ALLDGRVQL |
| 29 | CLASP1-001 | Similar peptides | RLLDGAFKL |
| 30 | COL6A1-001 | Similar peptides | ILLDGSASV |
| 31 | COL6A2-001 | Similar peptides | FLLDGSERL |
| 32 | COL6A3-006 | Similar peptides | FLFDGSANLV |
| 33 | COL6A3-008 | Similar peptides | FLFDGSANL |
| 34 | COL6A3-014 | Similar peptides | FLLDGSEGV |
| 35 | VWA2-001 | Similar peptides | FLLDGSNSV |
| 36 | VWF-001 | Similar peptides | FLLDGSSRL |
| 37 | CDRbl mutant 1 | Beta chain - CDR1 variant 1 | ARWHNN |
| 38 | CDRbl mutant 2 | Beta chain - CDR1 variant 2 | AKDHLN |
| 39 | CDRbl mutant 3 | Beta chain - CDR1 variant 3 | ARWHRN |
| 40 | CDRbl mutant 4 | Beta chain - CDR1 variant 4 | AMDHPY |
| 41 | CDRbl mutant 5 | Beta chain - CDR1 variant 5 | ATDHYN |
| 42 | CDRbl mutant 6 | Beta chain - CDR1 variant 6 | ARYHTN |
| 43 | CDRbl mutant 7 | Beta chain - CDR1 variant 7 | APYHLN |
| 44 | CDRbl mutant 8 | Beta chain - CDR1 variant 8 | AKDHTN |
| 45 | CDRbl mutant 9 | Beta chain - CDR1 variant 9 | ARYHRN |
| 46 | CDRb1 mutant 10 | Beta chain - CDR1 variant 10 | ARWHSN |
| 47 | CDRbl mutant 11 | Beta chain - CDR1 variant 11 | ATDHYN |
| 48 | CDRbl mutant 12 | Beta chain - CDR1 variant 12 | RWGDLN |
| 49 | CDRbl mutant 13 | Beta chain - CDR1 variant 13 | ARDHLN |
| 50 | 75-1 | Fab heavy chain with stabilized scTv R4P3F9S | MKWVTFISLLFLFSSAYSEVQLVESGGGLVQPGGSLRLSC AASGYSFTGYTMNWVRQAPGKGLEWVALINPYKGVSTYNQ KFKDRFTISVDKSKNTAYLQMNSLRAEDTAVYYCARSGYY GDSDWYFDVWGQGTLVTVSSASTKGPSVFPLAPSSKSTSG GTAALGCLVKDYFPEPVTVSWNSGALTSGVHTFPAVLQSS GLYSLSSVVTVPSSSLGTQTYICNVNHKPSNTKVDKKVEP KSCDKTHTSPPSPAPPVAGQKEVEQNSGPLSVPEGAIASL NCTYSDRGSQSFFWYRQYSGKSPELIMSIYSNGDKEDGRF TAQLNKASQYVSLLIRDSQPSDSATYLCAAYSGAGSYQLT FGKGTKLSVIPNIQNGGGGSGGGGSGGGGSGGGGSGGGGS GVTQTPKHLITATGQRVTLRCSPRSGDLSVYWYKQSLDQG LQFLIQYYNGEERAKGNISERFSAQQFPDLHSELNLSSLE LGDSALYFCASSVESSYGYTFGSGTRLTVVEDLKN MKWVTFISLLFLFSSAYSEVQLVESGGGLVQPGGSLRLSC AASGYSFTGYTMNWVRQAPGKGLEWVALINPYKGVSTYNQ KFKDRFTISVDKSKNTAYLQMNSLRAEDTAVYYCARSGYY GDSDWYFDVWGQGTLVTVSSASTKGPSVFPLAPSSKSTSG GTAALGCLVKDYFPEPVTVSWNSGALTSGVHTFPAVLQSS GLYSLSSVVTVPSSSLGTQTYICNVNHKPSNTKVDKKVEP KSCDKTHTSPPSPAPPVAG |
| 51 | 75- Fab heavy chain | Fab heavy chain | LGDSALTTCASSVLSSTGTTTGSGTRLTVVLDLRN MKWVTFISLLFLFSSAYSEVQLVESGGGLVQPGGSLRLSC AASGYSFTGYTMNWVRQAPGKGLEWVALINPYKGVSTYNQ KFKDRFTISVDKSKNTAYLQMNSLRAEDTAVYYCARSGYY GDSDWYFDVWGQGTLVTVSSASTKGPSVFPLAPSSKSTSG GTAALGCLVKDYFPEPVTVSWNSGALTSGVHTFPAVLQSS GLYSLSSVVTVPSSSLGTQTYICNVNHKPSNTKVDKKVEP KSCDKTHTSPPSPAPPVAG |
| 52 | 75- Fab light chain | Fab light chain | MKWVTFISLLFLFSSAYSDIQMTQSPSSLSASVGDRVTIT CRASQDIRNYLNWYQQKPGKAPKLLIYYTSRLESGVPSRF SGSGSGTDYTLTISSLQPEDFATYYCQQGNTLPWTFGQGT KVEIKRTVAAPSVFIFPPSDEQLKSGTASVVCLLNNFYPR EAKVQWKVDNALQSGNSQESVTEQDSKDSTYSLSSTLTLS KADYEKHKVYACEVTHQGLSSPVTKSFNRGEC |
| 53 | 1G4 alpha | 1G4 TCR alpha chain - full length | MKWVTFISLLFLFSSAYSDIQMTQSPSSLSASVGDRVTIT CRASQDIRNYLNWYQQKPGKAPKLLIYYTSRLESGVPSRF SGSGSGTDYTLTISSLQPEDFATYYCQQGNTLPWTFGQGT KVEIKRTVAAPSVFIFPPSDEQLKSGTASVVCLLNNFYPR EAKVQWKVDNALQSGNSQESVTEQDSKDSTYSLSSTLTLS KADYEKHKVYACEVTHQGLSSPVTKSFNRGEC METLLGLLILWLQLQWVSSKQEVTQIPAALSVPEGENLVL NCSFTDSAIYNLQWFRQDPGKGLTSLLLIQSSQREQTSGR LNASLDKSSGRSTLYIAASQPGDSATYLCAVRPTSGGSYI PTFGRGTSLIVHPYIQNPDPAVYQLRDSKSSDKSVCLFTD FDSQTNVSQSKDSDVYITDKTVLDMRSMDFKSNSAVAWSN KSDFACANAFNNSIIPEDTFFPSPESSCDVKLVEKSFETD TNLNFQNLSVIGFRILLLKVAGFNLLMTLRLWSS |
| 54 | 1G4 alpha leader | 1G4 TCR alpha chain - leader peptide | METLLGLLILWLQLQWVSSK |
| 55 | 1G4 alpha variable | 1G4 TCR alpha chain - variable domain | QEVTQIPAALSVPEGENLVLNCSFTDSAIYNLQWFRQDPG KGLTSLLLIQSSQREQTSGRLNASLDKSSGRSTLYIAASQ PGDSATYLCAVRPTSGGSYIPTFGRGTSLIVHP |
| 56 | 1G4 alpha constant | 1G4 TCR alpha chain - constant domain | YIQNPDPAVYQLRDSKSSDKSVCLFTDFDSQTNVSQSKDS DVYITDKTVLDMRSMDFKSNSAVAWSNKSDFACANAFNNS IIPEDTFFPSPESSCDVKLVEKSFETDTNLNFQNLSVIGF RILLLKVAGFNLLMTLRLWSS |
| 57 | 1G4 beta | 1G4 TCR beta chain - full length | MSIGLLCCAALSLLWAGPVNAGVTQTPKFQVLKTGQSMTL QCAQDMNHEYMSWYRQDPGMGLRLIHYSVGAGITDQGEVP NGYNVSRSTTEDFPLRLLSAAPSQTSVYFCASSYVGNTGE LFFGEGSRLTVLEDLKNVFPPEVAVFEPSEAEISHTQKAT LVCLATGFYPDHVELSWWVNGKEVHSGVSTDPQPLKEQPA LNDSRYCLSSRLRVSATFWQNPRNHFRCQVQFYGLSENDE WTQDRAKPVTQIVSAEAWGRADCGFTSESYQQGVLSATIL YEILLGKATLYAVLVSALVLMAMVKRKDSRG |
| 58 | 1G4 beta leader | 1G4 TCR beta chain - leader peptide | MSIGLLCCAALSLLWAGPVNA |
| 59 | 1G4 beta variable | 1G4 TCR beta chain - variable domain | GVTQTPKFQVLKTGQSMTLQCAQDMNHEYMSWYRQDPGMG LRLIHYSVGAGITDQGEVPNGYNVSRSTTEDFPLRLLSAA PSQTSVYFCASSYVGNTGELFFGEGSRLTVL |
| 60 | 1G4 beta constant | Beta chain - constant domain | GVTQTPKFQVLKTGQSMTLQCAQDMNHEYMSWYRQDPGMG LRLIHYSVGAGITDQGEVPNGYNVSRSTTEDFPLRLLSAA PSQTSVYFCASSYVGNTGELFFGEGSRLTVL EDLKNVFPPEVAVFEPSEAEISHTQKATLVCLATGFYPDH VELSWWVNGKEVHSGVSTDPQPLKEQPALNDSRYCLSSRL RVSATFWQNPRNHFRCQVQFYGLSENDEWTQDRAKPVTQI VSAEAWGRADCGFTSESYQQGVLSATILYEILLGKATLYA VLVSALVLMAMVKRKDSRG |
| 61 | NYESO1-001 | Control peptide | SLLMWITQV |
| 62 | C-14 beta; C-5 beta | C-14; C-5 TCR full length beta chain with CDRb1 mutant 4 | MGFRLLCCVAFCLLGAGPVDSGVTQTPKHLITATGQRVTL RCSPAMDHPYVYWYQQSLDQGLQFLIQYYNGEERAKGNIL ERFSAQQFPDLHSELNLSSLELGDSALYFCASSVESSYGY TFGSGTRLTVVEDLNKVFPPEVAVFEPSEAEISHTQKATL VCLATGFFPDHVELSWWVNGKEVHSGVSTDPQPLKEQPAL NDSRYCLSSRLRVSATFWQNPRNHFRCQVQFYGLSENDEW TQDRAKPVTQIVSAEAWGRADCGFTSVSYQQGVLSATILY EILLGKATLYAVLVSALVLMAMVKRKDF |
| 63 | C-14 alpha | C-14 TCR full length alpha chain with CDRal mutant 1 | MKSLRVLLVILWLQLSWVWSQQKEVEQNSGPLSVPEGAIA SLNCTYSDRRSQSFFWYRQYSGKSPELIMFIYSNGDKEDG RFTAQLNKASQYVSLLIRDSQPSDSATYLCAAYSGAGSYQ LTFGKGTKLSVIPNIQNPDPAVYQLRDSKSSDKSVCLFTD FDSQTNVSQSKDSDVYITDKTVLDMRSMDFKSNSAVAWSN KSDFACANAFNNSIIPEDTFFPSPESSCDVKLVEKSFETD TNLNFQNLSVIGFRILLLKVAGFNLLMTLRLWSS |
| 64 | 75-5 | Fab heavy chain with stabilized scTv R4P3F9S and CDRbl mutant 4 | MKWVTFISLLFLFSSAYSEVQLVESGGGLVQPGGSLRLSC AASGYSFTGYTMNWVRQAPGKGLEWVALINPYKGVSTYNQ KFKDRFTISVDKSKNTAYLQMNSLRAEDTAVYYCARSGYY GDSDWYFDVWGQGTLVTVSSASTKGPSVFPLAPSSKSTSG GTAALGCLVKDYFPEPVTVSWNSGALTSGVHTFPAVLQSS GLYSLSSVVTVPSSSLGTQTYICNVNHKPSNTKVDKKVEP KSCDKTHTSPPSPAPPVAGQKEVEQNSGPLSVPEGAIASL NCTYSDRGSQSFFWYRQYSGKSPELIMSIYSNGDKEDGRF TAQLNKASQYVSLLIRDSQPSDSATYLCAAYSGAGSYQLT FGKGTKLSVIPNIQNGGGGSGGGGSGGGGSGGGGSGGGGS GVTQTPKHLITATGQRVTLRCSPAMDHPYVYWYKQSLDQG LQFLIQYYNGEERAKGNISERFSAQQFPDLHSELNLSSLE LGDSALYFCASSVESSYGYTFGSGTRLTVVEDLKN |
| 65 | 75-14 | Fab heavy chain with stabilized scTv R4P3F9S, CDRal mutant 1 and CDRbl mutant 4 | MKWVTFISLLFLFSSAYSEVQLVESGGGLVQPGGSLRLSC AASGYSFTGYTMNWVRQAPGKGLEWVALINPYKGVSTYNQ KFKDRFTISVDKSKNTAYLQMNSLRAEDTAVYYCARSGYY GDSDWYFDVWGQGTLVTVSSASTKGPSVFPLAPSSKSTSG GTAALGCLVKDYFPEPVTVSWNSGALTSGVHTFPAVLQSS GLYSLSSVVTVPSSSLGTQTYICNVNHKPSNTKVDKKVEP KSCDKTHTSPPSPAPPVAGQKEVEQNSGPLSVPEGAIASL NCTYSDRRSQSFFWYRQYSGKSPELIMSIYSNGDKEDGRF TAQLNKASQYVSLLIRDSQPSDSATYLCAAYSGAGSYQLT FGKGTKLSVIPNIQNGGGGSGGGGSGGGGSGGGGSGGGGS GVTQTPKHLITATGQRVTLRCSPAMDHPYVYWYKQSLDQG LQFLIQYYNGEERAKGNISERFSAQQFPDLHSELNLSSLE LGDSALYFCASSVESSYGYTFGSGTRLTVVEDLKN |
| 66 | 75-25 | Fab heavy chain with stabilized scTv R4P3F9S in beta/alpha orientatio n, CDRal mutant 1 | MKWVTFISLLFLFSSAYSEVQLVESGGGLVQPGGSLRLSC AASGYSFTGYTMNWVRQAPGKGLEWVALINPYKGVSTYNQ KFKDRFTISVDKSKNTAYLQMNSLRAEDTAVYYCARSGYY GDSDWYFDVWGQGTLVTVSSASTKGPSVFPLAPSSKSTSG GTAALGCLVKDYFPEPVTVSWNSGALTSGVHTFPAVLQSS GLYSLSSVVTVPSSSLGTQTYICNVNHKPSNTKVDKKVEP KSCDKTHTSPPSPAPPVAGGVTQTPKHLITATGQRVTLRC SPRSGDLSVYWYKQSLDQGLQFLIQYYNGEERAKGNISER FSAQQFPDLHSELNLSSLELGDSALYFCASSVESSYGYTF GSGTRLTVVEDLKNGGGGSGGGGSGGGGSGGGGSGGGGSQ KEVEQNSGPLSVPEGAIASLNCTYSDRRSQSFFWYRQYSG KSPELIMSIYSNGDKEDGRFTAQLNKASQYVSLLIRDSQP SDSATYLCAAYSGAGSYQLTFGKGTKLSVIPNIQN |

### EXAMPLES

Native T cell receptors (TCRs) against cancer antigens are often of lower affinity when compared to TCRs targeting viral antigens, and this may be one possible explanation for tumor immune escape (Aleksic et al. 2012). Therefore, it is desirable to have higher affinity TCR variants designed for the use as antigen recognizing constructs in an adoptive cell therapy, or as recognition module of a soluble approach, i.e. using bispecific molecules (Hickman et al. 2016). This invention thus relates to the modification and optimization of the naturally occurring T cell receptor R4P3F9 (SEQ ID NOs: 2 and 10) targeting the tumor associated peptide COL6A3-002 (SEQ ID NO: 1) with an affinity of about 60 µM (DE102016115246).

### Example 1: Generation of stable scTv

For the present invention, the previously investigated TCR R4P3F9 (SEQ ID NOs: 2 and 10) was converted into a single chain TCR construct (scTv, SEQ ID NO: 22) for maturation via yeast surface display by combination of the variable alpha (SEQ ID NO: 4) and beta (SEQ ID NO: 12) domain with appendages of the respective constant domains (SEQ ID NOs: 9 and 17) and an appropriate glycine-serine linker sequence. The DNA of the corresponding sequence was synthesized and transformed into Saccharomyces cerevisiae EBY100 (MATa AGA1::GAL1AGA1::URA3 ura352 trp1 leu2delta200 his3-delta200 pep4::HIS3 prbd1.6R can1 GAL) (ATCC^{®} MYA 4941^{™}) together with a yeast display vector containing a leader sequence and the Aga2p yeast mating protein (SEQ ID NO: 20), based on pCT302 (Boder et al. 2000). The resulting fusion protein after homologous recombination in the yeast (SEQ ID NO: 19) contains a leader peptide at the N-terminus of the Aga2p protein, responsible for the display of the protein of interest (Boder et al. 1997), short peptide tags including linker sequences (SEQ ID NOs: 21 and 23) for expression controls and the protein of interest, namely the scTv R4P3F9 (SEQ ID NO: 22) or its variants. The transformation was performed as described in DE102016121899 and resulted in up to 10⁹ yeast clones per library. The libraries were generated via a random mutation PCR approach spanning the whole gene sequence of the scTv R4P3F9.

The selection process for the yeast clones bearing the best expressing scTv that is selectively binding to COL6A3-002 in context of HLA-A^{∗}02 was essentially performed as described in Smith et al 2015. In order to ascertain high expression and correct conformation of the scTv R4P3F9 variant, displayed on the yeast surface, an anti-Vbetal (Beckman Coulter, clone BL37.2) antibody was used, together with HLA-A^{∗}02/COL6A3-002 tetramer (Figure 1). The scTv conversion by yeast surface display revealed two crucial stabilizing mutations in the framework region together with the original CDR sequences for the proper presentation of the scTv on the cell surface, namely bQ43K (SEQ ID NO: 24) and bL72S (SEQ ID NO: 25), both located on the beta chain. Furthermore, during stability maturation position 29 in the CDR1 of the alpha chain (SEQ ID NO: 5) was converted from glycine to arginine (CDRal mutant 1, SEQ ID NO: 26), which resulted in improved tetramer binding.

### Example 2: Affinity maturation of stabilized scTv

To generate scTv molecules with higher binding affinity towards HLA-A^{∗}02/COL6A3-002, the CDRbl (SEQ ID NO: 13) was degenerated using the previously identified stabilized scTv R4P3F9S scaffold (SEQ ID NO: 27) expressing the stabilizing mutations aG29R, bQ43K and bL72S. The CDRbl residues were randomized by using degenerate DNA oligo primers essentially as described previously (Smith et al. 2015). The resulting DNA library was transformed as described in example 1. To retain scTv binding selectivity, negative selection was employed against HLA-A^{∗}02 tetramers comprising peptides derived from normal tissues (SEQ ID NOs: 28 to 36), which show high sequence similarity to COL6A3-002 peptide.

For the selection of affinity enhanced and selective scTv R4P3F9S variants a decreasing concentration of HLA-A^{∗}02/COL6A3-002 tetramer was used for each sorting round. After three selection rounds, single scTv clones were isolated and sequenced, resulting in a variety of affinity maturated CDRbl sequences (SEQ ID NOs: 37 to 49). For scTv with maturated CDRbl sequences a strong improvement in COL6A3-002 binding could be demonstrated while the selectivity of COL6A3-002 binding was retained as no binding of 9 similar peptides was observed (Figure 2).

### Example 3: Production of bispecific antibody-scTv fusion proteins

Stabilized and affinity maturated scTv against HLA-A^{∗}02/COL6A3-002 can be expressed in fusion with an antibody moiety directed against CD3 allowing tumor-specific retargeting and activation of T cells independent of their natural specificity. The inventors generated bispecific antibody-TCR fusion proteins comprising an anti-CD3 Fab (UCHT1) heavy chain (SEQ ID NO: 51) fused to scTv R4P3F9S variants (SEQ ID NOs: 50, 64, 65 and 66) and an anti-CD3 Fab (UCHT1) light chain (SEQ ID NO: 52). The resulting Fab-scTv fusion proteins have a molecular mass of approximately 75 kDa. Based on different CDR1 sequences of the scTv R4P3F9S alpha (SEQ ID NOs: 5 and 26) and beta chain (SEQ ID NOs: 13 and 37 to 49) different Fab-scTv fusion variants (75-1 to 75-25, Table 2) were expressed in transiently transfected ExpiCHO cells as recommended by the manufacturer. Proteins were purified by protein L and size exclusion chromatography. All fusion variants could be produced with yields ranging from 80 µg up to 1 mg (Table 2) and homogeneously formed heterodimers at the expected size as analyzed by size exclusion chromatography (Figure 3).

**Table 2: Nomenclature and yields of bispecific Fab-scTv fusion proteins. The molecules are based on SEQ ID NOs 50 and 52 and the indicated CDRal and CDRbl variants.**

| **Variant** | **CDRa1/SEQ** | **CDRb1/SEQ** | **Yield [µg]** |
|---|---|---|---|
| 75-1 | DRGSQS (SEQ ID NO. 5) | RSGDLS (SEQ ID NO. 13) | 267.9 |
| 75-2 | DRGSQS (SEQ ID NO. 5) | ARWHNN (SEQ ID NO. 37) | 78.4 |
| 75-3 | DRGSQS (SEQ ID NO. 5) | AKDHLN (SEQ ID NO. 38) | 646.7 |
| 75-4 | DRGSQS (SEQ ID NO. 5) | ARWHRN (SEQ ID NO. 39) | 704.3 |
| 75-5 | DRGSQS (SEQ ID NO. 5) | AMDHPY (SEQ ID NO. 40) | 397.2 |
| 75-6 | DRGSQS (SEQ ID NO. 5) | ATDHYN (SEQ ID NO. 41) | 268.1 |
| 75-7 | DRGSQS (SEQ ID NO. 5) | ARYHTN (SEQ ID NO. 42) | 83.2 |
| 75-8 | DRGSQS (SEQ ID NO. 5) | APYHLN (SEQ ID NO. 43) | 765.7 |
| 75-9 | DRGSQS (SEQ ID NO. 5) | AKDHTN (SEQ ID NO. 44) | 1067.2 |
| 75-10 | DRRSQS (SEQ ID NO. 26) | RSGDLS (SEQ ID NO. 13) | 389.6 |
| 75-11 | DRRSQS (SEQ ID NO. 26) | ARWHNN (SEQ ID NO. 37) | 270.4 |
| 75-12 | DRRSQS (SEQ ID NO. 26) | AKDHLN (SEQ ID NO. 38) | 943.6 |
| 75-13 | DRRSQS (SEQ ID NO. 26) | ARWHRN (SEQ ID NO. 39) | 560.3 |
| 75-14 | DRRSQS (SEQ ID NO. 26) | AMDHPY (SEQ ID NO. 40) | 360.7 |
| 75-15 | DRRSQS (SEQ ID NO. 26) | ATDHYN (SEQ ID NO. 41) | 541.5 |
| 75-16 | DRRSQS (SEQ ID NO. 26) | ARYHTN (SEQ ID NO. 42) | 403.6 |
| 75-17 | DRRSQS (SEQ ID NO. 26) | APYHLN (SEQ ID NO. 43) | 195.5 |
| 75-18 | DRRSQS (SEQ ID NO. 26) | AKDHTN (SEQ ID NO. 44) | 731.3 |
| 75-19 | DRRSQS (SEQ ID NO. 26) | ARYHRN (SEQ ID NO. 45) | 794 |
| 75-20 | DRRSQS (SEQ ID NO. 26) | ARWHSN (SEQ ID NO. 46) | 85.5 |
| 75-21 | DRRSQS (SEQ ID NO. 26) | ATDHYN (SEQ ID NO. 47) | 276 |
| 75-22 | DRRSQS (SEQ ID NO. 26) | RWGDLN (SEQ ID NO. 48) | 255 |
| 75-23 | DRRSQS (SEQ ID NO. 26) | ARDHLN (SEQ ID NO. 49) | 217 |
| 75-24^{a} | DRGSQS (SEQ ID NO: 5) | RSGDLS (SEQ ID NO. 13) | 166.6 |
| 75-25^{a} | DRRSQS (SEQ ID NO. 26) | RSGDLS (SEQ ID NO. 13) | 267 |

| | | | |
|---|---|---|---|
| ^{a} beta-alpha orientation of scTv | | | |

### Example 4: Fab-scTv fusion protein binding to COL6A3-002 and similar peptides

Binding affinity of anti-CD3-scTv R4P3F9S fusion proteins towards HLA-A^{∗}02 monomers with COL6A3-002 or different similar peptides was measured by biolayer interferometry. Measurements were done on an Octet RED384 system using settings recommended by the manufacturer. Briefly, purified Fab-scTv molecules were loaded onto biosensors (FAB2G) prior to analyzing serial dilutions of HLA-A^{∗}02/COL6A3-002. Compared to variants 75-1 and 75-24 comprising wild-type CDRal and wild-type CDRb1 increased binding affinities of up to 40-fold were observed for Fab-scTv variants with maturated CDRal and/or CDRb1 sequences (Table 3, Figure 4). In order to evaluate the selectivity of binding to HLA-A^{∗}02/COL6A3-002, purified Fab-scTv molecules loaded onto FAB2G biosensors were screened for binding to 1 µM similar peptides (SEQ ID NOs: 28 to 36), each in complex with HLA-A^{∗}02. Except of HLA-A^{∗}02/COL6A1-001 (SEQ ID NO: 30), which was bound by most of the Fab-scTv variants containing maturated CDRal (SEQ ID NO: 26), Fab-scTv variants showed no binding to similar peptides (Figure 5) arguing for high binding selectivity. For some Fab-scTv variants the therapeutic window between HLA-A^{∗}02/COL6A3-002 and HLA-A^{∗}02/COL6A1-001 binding was investigated by loading biotinylated peptide-HLA complexes onto biosensors (SA) and analyzing dilution series of Fab-scTv variants. While variant 75-10 comprising maturated CDRal (SEQ ID NO: 26) and wild-type CDRbl (SEQ ID NO: 13) sequence showed 8-fold increased binding affinity to HLA-A^{∗}02/COL6A3-002 over HLA-A^{∗}02/COL6A1-001, an up to 57-fold increased binding affinity was detected for Fab-scTv variant 75-13 comprising a maturated CDRbl (SEQ ID NO: 39) arguing for an improvement in therapeutic window (Table 4, Figure 6).

**Table 3: Binding affinity of Fab-scTv fusion proteins to HLA-A^{∗}02/COL6A3-002.**

| **Variant** | **KD (M)** | **kon (1/Ms)** | **koff (1/s)** |
|---|---|---|---|
| 75-1 | 8.06E-06 | 1.01E+05 | 8.17E-01 |
| 75-2 | 3.69E-06 | 1.59E+05 | 5.86E-01 |
| 75-3 | 4.92E-06 | 9.71E+04 | 4.78E-01 |
| 75-4 | 5.76E-06 | 9.78E+04 | 5.63E-01 |
| 75-5 | 4.32E-04 | 2.21E+03 | 9.55E-01 |
| 75-6 | 1.13E-06 | 2.06E+05 | 2.32E-01 |
| 75-7 | 1.79E-06 | 1.93E+05 | 3.44E-01 |
| 75-8 | 3.45E-06 | 1.36E+05 | 4.69E-01 |
| 75-9 | 1.41E-05 | 6.02E+04 | 8.51E-01 |
| 75-10 | 1.78E-06 | 1.69E+05 | 3.01E-01 |
| 75-11 | 2.82E-07 | 4.16E+05 | 1.18E-01 |
| 75-12 | 3.74E-07 | 2.67E+05 | 1.00E-01 |
| 75-13 | 4.05E-07 | 3.28E+05 | 1.33E-01 |
| 75-14 | 3.10E-06 | 8.41E+04 | 2.61E-01 |
| 75-15 | 7.78E-07 | 2.33E+05 | 1.81E-01 |
| 75-16 | 5.87E-07 | 3.37E+05 | 1.98E-01 |
| 75-17 | 2.27E-07 | 3.62E+05 | 8.20E-02 |
| 75-18 | 1.93E-06 | 1.51E+05 | 2.91E-01 |
| 75-19 | 6.00E-07 | 2.96E+05 | 1.78E-01 |
| 75-20 | 5.31E-07 | 6.08E+05 | 3.23E-01 |
| 75-21 | 5.52E-07 | 2.72E+05 | 1.50E-01 |
| 75-22 | 8.22E-07 | 2.48E+05 | 2.04E-01 |
| 75-23 | 3.24E-07 | 3.18E+05 | 1.03E-01 |
| 75-24 | 5.20E-06 | 1.08E+05 | 5.62E-01 |
| 75-25 | 8.33E-06 | 6.23E+04 | 5.19E-01 |

**Table 4: Comparative binding affinity of Fab-scTv fusion proteins to HLA-A^{∗}02/COL6A3-002 and HLA-A^{∗}02/COL6A1-001.**

| **Variant** | **pHLA-A*02** | **KD (M)** | **KD_{COL6A1-001}/KD_{COL6A3-002}** |
|---|---|---|---|
| 75-10 | COL6A3-002 | 1.37E-05 | 8 |
| | COL6A1-001 | 1.08E-04 | |
| 75-11 | COL6A3-002 | 8.50E-07 | 8 |
| | COL6A1-001 | 6.46E-06 | |
| 75-12 | COL6A3-002 | 7.24E-07 | 12 |
| | COL6A1-001 | 8.98E-06 | |
| 75-13 | COL6A3-002 | 7.39E-07 | 57 |
| | COL6A1-001 | 4.23E-05 | |
| 75-17 | COL6A3-002 | 8.25E-07 | 9 |
| | COL6A1-001 | 7.10E-06 | |
| 75-23 | COL6A3-002 | 1.15E-06 | 22 |
| | COL6A1-001 | 2.55E-05 | |

### Example 5: Use of affinity-maturated TCRs for cellular expression

Modification of T cells to express TCRs recognizing a tumor-specific peptide-HLA is a promising alternative of redirecting T cells to cancer cells. As the usage of maturated CDR1 sequences could improve cell-bound TCRs against HLA-A^{∗}02/COL6A3-002, the identified CDRal and CDRbl mutant sequences were grafted onto the parental TCR R4P3F9 (SEQ ID NOs: 2 and 10). The resulting mutant TCR variants (C-1 to C-18, Table 5) were expressed in human CD8⁺ T cells after electroporation of respective mRNA generated by in vitro transcription of PCR-amplified DNA constructs. For control purpose, the 1G4 TCR (SEQ ID NOs: 53 and 57) against NYESO1-001 peptide (SEQ ID NO: 61) was expressed. After overnight incubation of RNA-electroporated CD8⁺ T cells, expression of introduced TCR variants was analyzed by staining with PE-labeled HLA-A^{∗}02/COL6A3-002 tetramers or HLA-A^{∗}02/NYESO1-001 tetramers. While the parental TCR R4P3F9 variant C-1 showed only minimal staining with HLA-A^{∗}02/COL6A3-002 tetramers, the R4P3F9 TCR variants C-2 to C-18 with maturated CDRal and/or CDRbl showed increased tetramer staining (Figure 7). Functional activation of CD8⁺ T cells (20,000 cells/well) expressing different maturated R4P3F9 TCR variants was investigated by determining levels of released IFN-gamma upon co-culture with T2 cells (20,000 cells/well) loaded with either a dilution series of COL6A3-002 (SEQ ID NO: 1) or 10 µM of COL6A3-002 and similar peptides (SEQ ID NOs: 28 to 36). Compared to the parental R4P3F9 TCR variant C-1, maturated TCR variants C-2 to C-18 showed increased IFN-gamma release with maximum levels reached already at lower peptide concentrations (Figure 8). As expected no IFN-gamma release was observed with T cells expressing no TCR or the 1G4 control TCR specific for NYESO1-001. To analyze the selectivity of COL6A3-002 recognition of the maturated R4P3F9 TCR variants, the IFN-gamma release in response to T2 cells loaded with different similar peptides (SEQ ID NOs: 28 to 36) was analyzed and revealed different selectivity profiles for the maturated R4P3F9 TCR variants. Most interestingly, the TCR variants C-5 (SEQ ID Nos 62 and 2) and C-14 (SEQ ID NOs: 62 and 63) comprising the same maturated CDRbl (SEQ ID NO: 40) did not show any cross-reactivity towards COL6A1-001 or other similar peptides (Figure 9) making affinity maturated R4P3F9 TCR variants C-5 and C14 most promising candidates for cellular TCR-based tumor targeting.

**Table 5: Nomenclature of cellular TCR variants. The molecules are based on SEQ ID NOs 2 and 10 and the indicated CDRal and CDRbl variants.**

| **Variant** | **CDRa**1 | **CDRb1** |
|---|---|---|
| C-1 | DRGSQS (SEQ ID NO. 5) | RSGDLS (SEQ ID NO. 13) |
| C-2 | DRGSQS (SEQ ID NO. 5) | ARWHNN (SEQ ID NO. 37) |
| C-3 | DRGSQS (SEQ ID NO. 5) | AKDHLN (SEQ ID NO. 38) |
| C-4 | DRGSQS (SEQ ID NO. 5) | ARWHRN (SEQ ID NO. 39) |
| C-5 | DRGSQS (SEQ ID NO. 5) | AMDHPY (SEQ ID NO. 40) |
| C-6 | DRGSQS (SEQ ID NO. 5) | ATDHYN (SEQ ID NO. 41) |
| C-7 | DRGSQS (SEQ ID NO. 5) | ARYHTN (SEQ ID NO. 42) |
| C-8 | DRGSQS (SEQ ID NO. 5) | APYHLN (SEQ ID NO. 43) |
| C-9 | DRGSQS (SEQ ID NO. 5) | AKDHTN (SEQ ID NO. 44) |
| C-10 | DRRSQS (SEQ ID NO. 26) | RSGDLS (SEQ ID NO. 13) |
| C-11 | DRRSQS (SEQ ID NO. 26) | ARWHNN (SEQ ID NO. 37) |
| C-12 | DRRSQS (SEQ ID NO. 26) | AKDHLN (SEQ ID NO. 38) |
| C-13 | DRRSQS (SEQ ID NO. 26) | ARWHRN (SEQ ID NO. 39) |
| C-14 | DRRSQS (SEQ ID NO. 26) | AMDHPY (SEQ ID NO. 40) |
| C-15 | DRRSQS (SEQ ID NO. 26) | ATDHYN (SEQ ID NO. 41) |
| C-16 | DRRSQS (SEQ ID NO. 26) | ARYHTN (SEQ ID NO. 42) |
| C-17 | DRRSQS (SEQ ID NO. 26) | APYHLN (SEQ ID NO. 43) |
| C-18 | DRRSQS (SEQ ID NO. 26) | AKDHTN (SEQ ID NO. 44) |

### Example 6: Window of COL6A3-002 and COL6A1-001 recognition of cellular TCR variants

Cellular expression and analysis of R4P3F9 variants was performed as described above. In accordance with previous experiments (Figure 7), staining of T cells expressing R4P3F9 TCR variants C-2 to C-18 with PE-labeled HLA-A^{∗}02/COL6A3-002 tetramers were increased compared to the parental TCR C-1. Additionally, TCR variants C-12 and C-17 showed binding to HLA-A^{∗}02/COL6A1-001 (Figure 10). Expression of all maturated R4P3F9 variants improved functional activation of CD8⁺ T cells in response to T2 cells loaded with a dilution series of COL6A3-002 (SEQ ID NO: 1) reaching 5- to 90-fold lower EC₅₀ values compared to the parental TCR C-1 (Figure 11, Table 6). The lowest EC₅₀ value was found for variant C-14. Again, TCR variants C-5 (SEQ ID NOs 62 and 2) and C-14 (SEQ ID NOs: 62 and 63) comprising the same maturated CDRbl (SEQ ID NO: 40) did not show any cross-reactivity towards COL6A1-001, while other variants showed strong recognition with EC₅₀ windows (COL6A3-002 vs. COL6A1-001) as low as factor 5.

**Table 6: EC₅₀ values [nM] of IFN-γ release of T cells expressing R4P3F9 variants after coculture with T2 cells loaded with COL6A3-002 or COL6A1-001.**

| **Variant** | **EC₅₀ COL6A3-002 [nM]** | **EC₅₀ COL6A1-001 [nM]** |
|---|---|---|
| C-1 | 2.51 | - |
| C-2 | 0.16 | - |
| C-3 | 0.14 | *871^{a}* |
| C-4 | 0.13 | - |
| C-5 | 0.15 | - |
| C-6 | 0.48 | - |
| C-7 | 0.29 | - |
| C-8 | 0.20 | 350 |
| C-9 | 0.55 | - |
| C-10 | 0.32 | 1.5 |
| C-11 | 0.32 | 8.2 |
| C-12 | 0.20 | 1.9 |
| C-13 | 0.23 | 9.7 |
| C-14 | 0.03 | - |
| C-15 | 0.31 | 69 |
| C-16 | 0.34 | 78 |
| C-17 | 0.33 | 4.1 |
| C-18 | 0.14 | *280089^{a}* |

| | | |
|---|---|---|
| *^{a} plateau not reached* | | |

### Example 7: Efficacy of maturated R4P3F9 variants C-5 and C-14 on tumor cell lines

Cellular expression and analysis of R4P3F9 variants was performed as described above. Expression of the maturated R4P3F9 variants C-5 (SEQ ID NOs 62 and 2) and C-14 (SEQ ID NOs: 62 and 63) improved functional activation of CD8⁺ T cells in response to COL6A3-002 (SEQ ID NO: 1)-presenting tumor cell lines as compared to the parental TCR C-1 (Figure 12). The tumor cell lines used during this study present different amounts of target peptide. SF539 cells carry ~4000 copies of HLA-A^{∗}02/COL6A3-002 per cell and SW982 cells carry ~460 copies per cell. Whereas the parental TCR C-1 did not mediate strong T-cell activation upon co-culture with target-positive cell lines, TCR variant C-14 showed even stronger improvement of functional activation than TCR variant C-5. These data are in line with EC₅₀ improvements from TCR C-1 to C-5 and to C-14 (table 6). The target-negative tumor cell line MCF-7 was not recognized by any of these TCRs.

### References:

Aleksic et al. 2012: Different affinity windows for virus and cancer-specific T-cell receptors - implications for therapeutic strategies, Eur J Immunol. 2012 Dec;42(12):3174-9;
Hickman et al. 2016: Antigen Selection for Enhanced Affinity T-Cell Receptor-Based Cancer Therapies, J Biomol Screen. 2016 Sep;21(8):769-85;
Boder and Wittrup 2000: Yeast surface display for directed evolution of protein expression, affinity, and stability, Methods Enzymol. 2000;328:430-44;
Boder and Wittrup 1997: Yeast surface display for screening combinatorial polypeptide libraries, Nat Biotechnol. 1997 Jun;15(6):553-7;
Smith et al. 2015: T Cell Receptor Engineering and Analysis Using the Yeast Display Platform, Methods Mol Biol. 2015;1319:95-141;
DE102016121899.5
DE102016115246

## Claims

1. An antigen recognizing construct comprising a first domain and a second domain, each domain comprising three complementary determining regions (CDRs), wherein the first domain comprises the amino acid sequences according to SEQ ID NOs: 6 (CDRa2) and 7 (CDRa3), and the second domain comprises the amino acid sequences according to SEQ ID NOs: 14 (CDRb2) and 15 (CDRb3), wherein
a) the CDRal of the first domain is as set forth in SEQ ID NO:5 and the CDRbl of the second domain is selected from one of the amino acid sequences according to SEQ ID NOs: 37 to 44, or
b) the CDRal of the first domain is as set forth in SEQ ID NO:26 and the CDRbl of the second domain is selected from one of the amino acid sequences according to SEQ ID NOs: 13 and 37 to 49, wherein the antigen recognizing construct is a T cell receptor (TCR) or derivative or fragment thereof, wherein the derivative or fragment retains the antigen binding and/or recognizing ability of the parent molecule to specifically and/or selectively bind to a COL6A3 antigenic polypeptide.

2. The antigen recognizing construct according to claim 1, wherein said antigen recognizing construct is stable, and capable of specifically and/or selectively binding to a COL6A3 antigenic peptide comprising the amino acid sequence according to SEQ ID NO: 1, in particular in the context with MHC.

3. The antigen recognizing construct according to any one of claims 1 to 2, wherein said first domain is part of a TCR α or γ chain; and/or wherein said second domain is part of a TCR β or δ chain.

4. The antigen recognizing construct according to any one of claims 1 to 3, wherein the antigen recognizing construct is a single chain antigen recognizing construct, preferably wherein the single chain antigen recognizing construct is a single chain TCR.

5. The antigen recognizing construct according to any one of claims 1 to 4, wherein the antigen recognizing construct is a soluble TCR.

6. A nucleic acid encoding for an antigen recognizing construct according to any one of claims 1 to 5.

7. A nucleic acid vector comprising the nucleic acid of claim 6.

8. A recombinant host cell comprising an antigen recognizing construct according to any one of claims 1 to 5, the nucleic acid of claim 6 or the vector according to claim 7, wherein said host cell preferably is a mammalian cell, more preferably a human cell.

9. The host cell according to claim 8, wherein said host cell is
a) a lymphocyte, preferably a T lymphocyte or T lymphocyte progenitor cell, more preferably a CD4 or CD8 positive T cell, or
b) a non-lymphocyte.

10. A pharmaceutical composition comprising the antigen recognizing construct according to any one of claims 1 to 5, the nucleic acid according to claim 6, the vector according to claim 7, or the host cell according to claim 8 or 9, and a pharmaceutically acceptable carrier, diluent stabilizer and/or excipient.

11. A method of producing the COL6A3-specific antigen recognizing construct according to any one of claims 1 to 5, comprising
a. providing a suitable host cell,
b. providing a genetic construct comprising a coding sequence encoding the antigen recognizing construct according to any of claims 1 to 5,
c. introducing said genetic construct into said suitable host cell, and
d. expressing said genetic construct by said suitable host cell, the method preferably further comprising isolating and purifying the antigen recognizing construct from the suitable host cell and, optionally, reconstituting the antigen recognizing construct in a T-cell.

12. The antigen recognizing construct according to any one of claims 1 to 5, the nucleic acid according to claim 6, the vector according to claim 7, the host cell according to claim 8 or 9, or the pharmaceutical composition according to claim 10, for use in medicine.

13. The antigen recognizing construct according to any one of claims 1 to 5, the nucleic acid according to claim 6, the vector according to claim 7, the host cell according to claim 8 or 9, or the pharmaceutical composition according to claim 10 for use in the diagnosis, prevention, and/or treatment of cancer, wherein said cancer is selected from a cancer where COL6A3 is overexpressed, mutated, and/or a COL6A3-derived tumor-associated antigen is presented.

14. An in vitro method for treating cancer or COL6A3-positive premalignancy, wherein said cancer is selected from a cancer where COL6A3 is overexpressed, mutated, and/or a COL6A3-derived tumor-associated antigen is presented, the method comprising:
a) transforming a cell obtained from a subject in need of a cancer treatment with at least one vector according to claim 7 to produce a transformed cell; and
b) expanding the transformed cell to produce a plurality of transformed cells.

15. An in vitro method for detecting cancer in a biological sample comprising:
a) contacting the biological sample with the antigen recognizing construct according to any one of claims 1 to 5;
b) detecting binding of the antigen recognizing construct to the biological sample,
wherein said cancer is selected from a cancer where COL6A3 is overexpressed, mutated, and/or a COL6A3-derived tumor-associated antigen is presented.

## Patentansprüche

1. Antigen-erkennendes Konstrukt, umfassend eine erste Domäne und eine zweite Domäne, wobei jede Domäne drei komplementaritätsbestimmende Regionen (CDRs) umfasst, wobei die erste Domäne die Aminosäuresequenzen gemäß SEQ ID NO: 6 (CDRa2) und 7 (CDRa3) umfasst, und die zweite Domäne die Aminosäuresequenzen gemäß SEQ ID NO: 14 (CDRb2) und 15 (CDRb3) umfasst, wobei
a) die CDRal der ersten Domäne in SEQ ID NO: 5 dargestellt ist und die CDRbl der zweiten Domäne aus einer der Aminosäuresequenzen gemäß SEQ ID NO: 37 bis 44 ausgewählt ist, oder
b) die CDRal der ersten Domäne in SEQ ID NO: 26 dargestellt ist und die CDRbl der zweiten Domäne aus einer der Aminosäuresequenzen gemäß SEQ ID NO: 13 und 37 bis 49 ausgewählt ist, wobei das Antigen-erkennende Konstrukt ein T-Zellrezeptor (TCR) oder ein Derivat oder ein Fragment davon ist, wobei das Derivat oder das Fragment die Antigenbindungs- und/oder Antigenerkennungsfähigkeit des Stammmoleküls, spezifisch und/oder selektiv an ein COL6A3-Antigenpeptid zu binden, behält.

2. Antigen-erkennendes Konstrukt nach Anspruch 1, wobei das Antigen-erkennende Konstrukt stabil ist und in der Lage ist, spezifisch und/oder selektiv an ein COL6A3-Antigenpeptid zu binden, das die Aminosäuresequenz gemäß SEQ ID NO: 1 umfasst, insbesondere im Zusammenhang mit MHC.

3. Antigen-erkennendes Konstrukt nach einem der Ansprüche 1 bis 2, wobei die erste Domäne Teil einer TCR α- oder γ-Kette ist; und/oder wobei die zweite Domäne Teil einer TCR β- oder δ-Kette ist.

4. Antigen-erkennendes Konstrukt nach einem der Ansprüche 1 bis 3, wobei das Antigen-erkennende Konstrukt ein einzelkettiges Antigen-erkennendes Konstrukt ist, bevorzugt wobei das einzelkettige Antigen-erkennende Konstrukt ein einzelkettiger TCR ist.

5. Antigen-erkennendes Konstrukt nach einem der Ansprüche 1 bis 4, wobei das Antigen-erkennende Konstrukt ein löslicher TCR ist.

6. Nukleinsäure, die ein Antigen-erkennendes Konstrukt nach einem der Ansprüche 1 bis 5 kodiert.

7. Nukleinsäure-Vektor, umfassend die Nukleinsäure nach Anspruch 6.

8. Rekombinante Wirtszelle umfassend ein Antigen-erkennendes Konstrukt nach einem der Ansprüche 1 bis 5, die Nukleinsäure nach Anspruch 6 oder den Vektor nach Anspruch 7, wobei die Wirtszelle bevorzugt eine Säugetierzelle, weiter bevorzugt eine menschliche Zelle ist.

9. Wirtszelle nach Anspruch 8, wobei die Wirtszelle
a) ein Lymphozyt, bevorzugt ein T-Lymphozyt oder T-Lymphozytvorläufer, weiter bevorzugt eine CD4- oder CD8-positive T-Zelle ist, oder
b) ein Nicht-Lymphozyt ist.

10. Pharmazeutische Zusammensetzung umfassend ein Antigen-erkennendes Konstrukt nach einem der Ansprüche 1 bis 5, die Nukleinsäure nach Anspruch 6 den Vektor nach Anspruch 7 oder die Wirtszelle nach Anspruch 8 oder 9 und einen pharmazeutisch akzeptablen Träger, Verdünnungsmittel, Stabilisator und/oder Hilfsstoff.

11. Verfahren zur Herstellung des COL6A3-spezifischen Antigen-erkennenden Konstrukts nach einem der Ansprüche 1 bis 5, umfassend
a. Bereitstellung einer geeigneten Wirtszelle,
b. Bereitstellung eines genetischen Konstrukts umfassend eine kodierende Sequenz, die das Antigen-erkennende Konstrukt nach einem der Ansprüche 1 bis 5 kodiert,
c. Einführung des genetischen Konstrukts in die geeignete Wirtszelle und
d. Expression des genetischen Konstrukts durch die geeignete Wirtszelle, wobei das Verfahren bevorzugt weiterhin die Isolierung und Aufreinigung des Antigen-erkennenden Konstrukts aus der geeigneten Wirtszelle und, optional, Rekonstitution des Antigen-erkennenden Konstrukts in eine T-Zelle umfasst.

12. Antigen-erkennendes Konstrukt nach einem der Ansprüche 1 bis 5, die Nukleinsäure nach Anspruch 6, der Vektor nach Anspruch 7, die Wirtszelle nach Anspruch 8 oder 9 oder die pharmazeutische Zusammensetzung nach Anspruch 10 zur Verwendung in der Medizin.

13. Antigen-erkennendes Konstrukt nach einem der Ansprüche 1 bis 5, die Nukleinsäure nach Anspruch 6, der Vektor nach Anspruch 7, die Wirtszelle nach Anspruch 8 oder 9 oder die pharmazeutische Zusammensetzung nach Anspruch 10 zur Verwendung in der Diagnose, Vorbeugung und/oder bei der Behandlung von Krebs, wobei der Krebs aus Krebsarten ausgewählt ist, bei welchen COL6A3 überexprimiert, mutiert ist und/oder ein vom COL6A3 abgeleitetes tumorassoziiertes Antigen präsentiert wird.

14. *In* vitro-Verfahren zur Behandlung von Krebs oder einer COL6A3-positiven Krankheitsvorstufe, wobei der Krebs aus Krebsarten ausgewählt ist, bei welchen COL6A3 überexprimiert, mutiert ist und/oder ein vom COL6A3 abgeleitetes tumorassoziiertes Antigen präsentiert wird, wobei das Verfahren umfasst:
a) Transformieren einer Zelle, die von einem Subjekt, das eine Krebsbehandlung benötigt, erhalten wird, mit mindestens einem Vektor nach Anspruch 7, um eine transformierte Zelle zu erzeugen; und
b) Vermehrung der transformierten Zelle, um eine Vielzahl von transformierten Zellen zu erzeugen.

15. *In* vitro-Verfahren zum Nachweis von Krebs in einer biologischen Probe umfassend:
a) Inkontaktbringen der biologischen Probe mit einem Antigen-erkennenden Konstrukt nach einem der Ansprüche 1 bis 5;
b) Nachweisen der Bindung des Antigen-erkennenden Konstrukts mit der biologischen Probe,
wobei der Krebs aus Krebsarten ausgewählt ist, bei welchen COL6A3 überexprimiert, mutiert ist und/oder ein vom COL6A3 abgeleitetes tumorassoziiertes Antigen präsentiert wird.

## Revendications

1. Construction reconnaissant des antigènes comprenant un premier domaine et un second domaine, chaque domaine comprenant trois régions déterminantes complémentaires (CDR), où le premier domaine comprend les séquences d'acides aminés conformes aux SEQ ID NO : 6 (CDRa2) et 7 (CDRa3), et le second domaine comprend les séquences d'acides aminés conformes aux SEQ ID NO : 14 (CDRb2) et 15 (CDRb3), où
a) la CDRal du premier domaine est telle que présentée dans la SEQ ID NO : 5 et la CDRbl du second domaine est sélectionnée parmi l'une des séquences d'acides aminés conformes aux SEQ ID NO : 37 à 44, ou
b) la CDRal du premier domaine est telle que présentée dans la SEQ ID NO : 26 et la CDRb1 du second domaine est sélectionnée parmi l'une des séquences d'acides aminés conformes aux SEQ ID NO : 13 et 37 à 49, où la construction reconnaissant des antigènes est un récepteur de lymphocytes T (TCR) ou un dérivé ou fragment de celui-ci, où le dérivé ou fragment conserve la capacité de reconnaissance et/ou de liaison à l'antigène de la molécule parente pour se lier spécifiquement et/ou sélectivement à un polypeptide antigénique COL6A3.

2. Construction reconnaissant des antigènes conforme à la revendication 1, où ladite construction reconnaissant des antigènes est stable, et capable de se lier spécifiquement et/ou sélectivement à un peptide antigénique COL6A3 comprenant la séquence d'acides aminés conforme à la SEQ ID n° 1, en particulier dans le contexte du CMH

3. Construction reconnaissant des antigènes conforme à l'une des revendications 1 à 2, où ledit premier domaine fait partie d'une chaîne TCR α ou γ ; et/ou où ledit second domaine fait partie d'une chaîne TCR β ou δ.

4. Construction reconnaissant des antigènes conforme à l'une des revendications 1 à 3, où la construction reconnaissant des antigènes est une construction reconnaissant des antigènes à chaîne unique, de préférence où la construction reconnaissant des antigènes à chaîne unique est un TCR à chaîne unique.

5. Construction reconnaissant des antigènes conforme à l'une des revendications 1 à 4, où la construction reconnaissant des antigènes est un TCR soluble.

6. Acide nucléique codant pour une construction reconnaissant des antigènes conforme à l'une des revendications 1 à 5.

7. Vecteur d'acide nucléique comprenant l'acide nucléique de la revendication 6.

8. Cellule hôte recombinante comprenant une construction reconnaissant des antigènes conforme à l'une des revendications 1 à 5, l'acide nucléique de la revendication 6 ou le vecteur conforme à la revendication 7, où ladite cellule hôte est de préférence une cellule de mammifère, plus préférablement une cellule humaine.

9. Cellule hôte conforme à la revendication 8, où ladite cellule hôte est
a) un lymphocyte, de préférence un lymphocyte T ou une cellule progénitrice de lymphocyte T, plus préférablement un lymphocyte T CD4-positif ou CD8-positif, ou
b) une cellule qui n'est pas un lymphocyte.

10. Composition pharmaceutique comprenant la construction reconnaissant des antigènes conforme à l'une des revendications 1 à 5, l'acide nucléique conforme à la revendication 6, le vecteur conforme à la revendication 7, ou la cellule hôte conforme à la revendication 8 ou 9, et un transporteur, diluant, stabilisant et/ou excipient pharmaceutiquement acceptables.

11. Procédé de production de la construction reconnaissant des antigènes spécifique à COL6A3 conforme à l'une des revendications 1 à 5, comprenant
a. la fourniture d'une cellule hôte appropriée,
b. la fourniture d'une construction génétique comprenant une séquence codante codant pour la construction reconnaissant des antigènes conforme à l'une des revendications 1 à 5,
c. l'introduction de ladite construction génétique dans ladite cellule hôte appropriée, et
d. l'expression de ladite construction génétique par ladite cellule hôte appropriée, le procédé comprenant également de préférence l'isolement et la purification de la construction reconnaissant des antigènes de la cellule hôte appropriée et, facultativement, la reconstitution de la construction reconnaissant des antigènes dans un lymphocyte T.

12. Construction reconnaissant des antigènes conforme à l'une des revendications 1 à 5, acide nucléique conforme à la revendication 6, vecteur conforme à la revendication 7, cellule hôte conforme à la revendication 8 ou 9 ou composition pharmaceutique conforme à la revendication 10, pour usage médical.

13. Construction reconnaissant des antigènes conforme à l'une des revendications 1 à 5, acide nucléique conforme à la revendication 6, vecteur conforme à la revendication 7, cellule hôte conforme à la revendication 8 ou 9 ou composition pharmaceutique conforme à la revendication 10, pour une utilisation dans le diagnostic, la prévention et/ou le traitement du cancer, où ledit cancer est sélectionné à partir d'un cancer dans lequel COL6A3 est surexprimé, muté, et/ou dans lequel un antigène associé à des tumeurs dérivé de COL6A3 est présenté.

14. Procédé *in vitro* pour traiter un cancer ou une maladie précancéreuse positive pour COL6A3, où ledit cancer est sélectionné à partir d'un cancer dans lequel COL6A3 est surexprimé, muté et/ou dans lequel un antigène associé à des tumeurs dérivé de COL6A3 est présenté, le procédé comprenant :
a) la transformation d'une cellule obtenue chez un sujet nécessitant un traitement anticancéreux avec au moins un vecteur conforme à la revendication 7 pour produire une cellule transformée ; et
b) l'amplification de la cellule transformée pour produire un grand nombre de cellules transformées.

15. Procédé *in vitro* pour la détection du cancer dans un échantillon biologique, comprenant :
a) la mise en contact de l'échantillon biologique avec la construction reconnaissant des antigènes conforme à l'une des revendications 1 à 5 ;
b) la détection de la liaison de la construction reconnaissant des antigènes à l'échantillon biologique,
où ledit cancer est sélectionné à partir d'un cancer dans lequel COL6A3 est surexprimé, muté et/ou dans lequel un antigène associé à des tumeurs dérivé de COL6A3 est présenté.
